# EUROPEAN PATENT SPECIFICATION

(11) **EP 3 813 870 B1**
(45) Date of publication and mention of the grant of the patent: **15.11.2023**
(21) Application number: 19746141.1
(22) Date of filing: 24.05.2019
(51) Int. Cl.: A61K 38/18, A61P 35/00

(54) **CCR5 INHIBITOR FOR USE IN TREATING CANCER**
CCR5-INHIBITOR ZUR VERWENDUNG BEI DER BEHANDLUNG VON KREBS
INHIBITEUR DE CCR5 DESTINÉ À ÊTRE UTILISÉ DANS LE TRAITEMENT DU CANCER

(30) Priority: 28.05.2018 US 201862677114 P; 15.01.2019 US 201962792600 P
(43) Date of publication of application: 05.05.2021
(73) Proprietor: Orion Biotechnology Switzerland Sàrl, 1202 Geneva (CH)
(72) Inventor: HARTLEY, Oliver, 1256 Troinex (CH); OFFORD, Robin Ewart, 1233 Bernex (CH)
(74) Representative: E. Blum & Co. AG
(86) International application number: PCT/IB2019/054350
(87) International publication number: WO 2019/229616

(56) References cited:
- US-B2- 8 686 111
- ROBINSON S C ET AL: "A CHEMOKINE RECEPTOR ANTAGONIST INHIBITS EXPERIMENTAL BREAST TUMOR GROWTH", CANCER RESEARCH, AMERICAN ASSOCIATION FOR CANCER RESEARCH, US, vol. 63, no. 23, 1 December 2003 (2003-12-01), pages 8360-8365, XP009057022, ISSN: 0008-5472
- A C BORCZUK ET AL: "Lung adenocarcinoma invasion in TGF[beta]RII-deficient cells is mediated by CCL5/RANTES", ONCOGENE, vol. 27, no. 4, 23 July 2007 (2007-07-23), pages 557-564, XP055625979, London ISSN: 0950-9232, DOI: 10.1038/sj.onc.1210662

## Description

### CROSS-REFERENCE TO RELATED APPLICATIONS

This application claims the benefit of and priority from United States Provisional Patent Application No. 62/677,114 filed on May 28, 2018 and United States Provisional Patent Application No. 62/792,600 filed on January 15, 2019.

### FIELD

The present application relates to a chemokine receptor inhibitor for use in methods of treating cancer.

### BACKGROUND

Chemokine and chemokine receptors have been implicated in a wide range of diseases. One chemokine can bind to several chemokine receptors and vice versa, so that they form a redundant system.

Among the known chemokine receptors, C-C chemokine receptor type 5 (CCR5) has been identified as a co-receptor that mediates HIV entry. CCR5 exists in at least two conformations at the cell surface, and among the possible explanations for their existence is one in which one conformation consists of G protein-coupled CCR5 and the other G protein-uncoupled CCR5. The C-C chemokines CCL3, CCL4 and CCL5 are the main CCR5 endogenous agonists. CCL5, also known as RANTES (regulated on activation, normal T cell expressed and secreted), has been shown to interact with C-C chemokine receptor type 1 (CCR1) and C-C chemokine receptor type 3 (CCR3) in addition to CCR5.

Engagement of endogenous CCR5 agonists leads to activation of a repertoire of intracellular signaling pathways, broadly defined as G protein-dependent and G protein-independent signaling pathways. One manifestation of G protein-dependent signaling is the induction of intracellular calcium flux. One manifestation of G protein-independent signaling is the induction of receptor internalization leading to intracellular receptor sequestration.

CCR5 belongs to the G protein-coupled receptor superfamily. For members of this superfamily, ligands capable of preferentially activating either G protein-dependent signaling pathways or G protein independent signaling pathways have been identified. These ligands, collectively known as 'biased ligands', have been shown to have interesting pharmacological and pharmaceutical attributes, being able to selectively activate or inhibit certain pathways of interest to the pathology.

CCR5 is expressed by certain cancer cells, where its activation is suggested to provide survival and metastatic signals. In addition, CCR5 is expressed on immunomodulatory leukocytes (M2 macrophages and regulatory T cells) that infiltrate the tumor and suppress anti-tumor immune responses.

Maraviroc (MVC) (Selzentry^{®}) is a CCR5 inhibitor that is currently approved for the treatment of HIV infection. In addition, maraviroc has also been shown to have activity in both *in vitro* (Pervaiz A. et al. Med Oncol. 2015;32(5): 158; Zi J. et al. Am J Cancer Res. 2017;7(4):869-80) and *in vivo* cancer models (Ward S.T. et al. Br J Cancer. 2015;112(2):319-28; Tanabe Y. et al. Oncotarget. 2016;7(30):48335-45).

In addition, patients with metastatic colorectal cancer (mCRC) who received oral maraviroc showed evidence of changes in the tumor microenvironment that might inhibit tumor growth or metastasis. In some cases, patients also experienced a clinical response to treatment with maraviroc that included regression of metastatic disease (Halama N. et al. Cancer Cell. 2016;29(4):587-601).

Robinson S C et al. ("A chemokine receptor antagonist inhibits experimental breast tumor growth", Cancer Research vol. 63, no. 23, 2003-12-01) describes anticancer activity of MET-RANTES (Met-CCL5) in a murine model of breast cancer.

Borczuk et al.: ("Lung adenocarcinoma invasion in TGF[beta]RII-deficient cells is mediated by CCL5/RANTES", ONCOGENE, vol. 27, no. 4, 23, 2007-07-23) suggests a role for CCR5 inhibition in lung adenocarcinoma prevention and treatment.

US 8,686,111 relates to fully coded polypeptide derivatives of a CCR5 inhibitor (RANTES) and uses thereof for the treatment of HIV infections. Said polypeptide derivatives comprise an N-terminal portion and a C-terminal portion, wherein said N-terminal portion comprises the signature sequence QGP[P or L] and the amino acid sequence of said C-terminal portion is at least 70% identical to SEQ ID NO: 1.

### SUMMARY

In one aspect, there is provided a CCR5 inhibitor for use in a method of treating cancer, said method comprising administering a CCR5 inhibitor to a subject, wherein the CCR5 inhibitor comprises an N-terminal portion and a C-terminal portion, wherein the N-terminal portion comprises the signature sequence QGP[P or L] and the amino acid sequence of the C-terminal portion is at least 70% identical to SEQ ID NO: 1 and wherein the CCR5 inhibitor inhibits HIV entry into cells and is selective for CCR5 over CCR1 and CCR3. The references to methods of treating and preventing cancer described herein are to be understood as references to said CCR5 inhibitor for use according to the present invention in those methods.

In an embodiment of the method as described herein, the CCR5 inhibitor inhibits only a subset of CCR5 intracellular signaling pathways.

In an embodiment of the method as described herein, the CCR5 inhibitor inhibits or reduces the inflammatory effects of CCR5, for example, leads to a signaling response of 30% or less of the maximum response (Emax) elicited by PSC-RANTES, when tested at a concentration of 300 nM in a Calcium Flux signaling assay.

In an embodiment of the method as described herein, the signature sequence is QGP[P or L] [L or G or S or M] [M or D or S or Q or G].

In an embodiment of the method as described herein, the signature sequence is QGP[P or L][L or G or S or M][M or D or S or Q or G]XX[Q or G or L or A or T or S]X, wherein X denotes any natural or modified amino acid.

In an embodiment of the method as described herein, the signature sequence is QGP[P or L]LM or QGPPG[D or S].

In an embodiment of the method as described herein, the signature sequence is QGPPLM or QGPPGD.

In an embodiment of the method as described herein, the signature sequence is QGP[P or L][L or M][M or Q][A or W or G or Q or N]X[Q or G or L][S or V or T or G], wherein X denotes any natural or modified amino acid.

In an embodiment of the method as described herein, the signature sequence is QGPPLM[A or W][L or T or M][Q or G][S or V or T or G].

In an embodiment of the method as described herein, the signature sequence is QGPP[G or L][M or Q]XX[Q or S][S or V], wherein X denotes any natural or modified amino acid.

In an embodiment of the method as described herein, the signature sequence is selected from the group QGPPLMALQS (SEQ ID NO: 2), QGPPLMWMQV (SEQ ID NO: 3), QGPPLMWLQV (SEQ ID NO: 4), QGPPLMWTQS (SEQ ID NO: 5), QGPPLMWLQT (SEQ ID NO: 6), QGPPLMWTQV (SEQ ID NO: 7), QGPPLMWMQS (SEQ ID NO: 8), QGPPLMATQS (SEQ ID NO: 9), QGPPLMWLQS (SEQ ID NO: 10), QGPPLMALQV (SEQ ID NO: 11), QGPPLMWLGG (SEQ ID NO: 12), QGPPLMWRGS (SEQ ID NO: 13), QGPLLMWLQV (SEQ ID NO: 14), QGPPLMQTTP (SEQ ID NO: 15), QGPPLSWLQV (SEQ ID NO: 30), QGPPLSWLQS (SEQ ID NO: 31), QGPPGQWSQV (SEQ ID NO: 32), QGPPMMAGLS (SEQ ID NO: 33), QGPPLSWQQS (SEQ ID NO: 34), QGPPGMWSQS (SEQ ID NO: 35), QGPPLQWRQS (SEQ ID NO: 36), QGPPLMGTQS (SEQ ID NO: 37), QGPPLMQLQV (SEQ ID NO: 38), QGPPLSWSQV (SEQ ID NO: 39), QGPPMSWSQS (SEQ ID NO: 40), QGPPLMNLQV (SEQ ID NO: 41), QGPPMSAYQV (SEQ ID NO: 42) and QGPPMQGGLS (SEQ ID NO: 43).

In an embodiment of the method as described herein, the signature sequence is selected from the group QGPPLMALQS (SEQ ID NO: 2), QGPPLMWMQV (SEQ ID NO: 3), QGPPLMWLQV (SEQ ID NO: 4), QGPPLMWTQS (SEQ ID NO: 5), QGPPLMWLQT (SEQ ID NO: 6), QGPPLMWTQV (SEQ ID NO: 7), QGPPLMWMQS (SEQ ID NO: 8), QGPPLMATQS (SEQ ID NO: 9), QGPPLMWLQS (SEQ ID NO: 10), QGPPLMALQV (SEQ ID NO: 11), QGPPLMWLGG (SEQ ID NO: 12), QGPPLMWRGS (SEQ ID NO: 13), QGPLLMWLQV (SEQ ID NO: 14) and QGPPLMQTTP (SEQ ID NO: 15).

In an embodiment of the method as described herein, the signature sequence is QGPPLMATQS (SEQ ID NO: 9).

In an embodiment of the method as described herein, the signature sequence is located at the extreme N-terminus.

In an embodiment of the method as described herein, the C-terminal portion is identical to SEQ ID NO: 1.

In an embodiment of the method as described herein, wherein the CCR5 inhibitor comprises the amino acid sequence of SEQ ID NO: 70.

In an embodiment of the method as described herein, the cancer is colorectal cancer, breast cancer, lung cancer, prostate cancer, ovarian cancer, pancreatic cancer, esophageal cancer, gastric cancer, liver cancer, or leukemia.

In an embodiment of the method as described herein, wherein the cancer is metastatic.

In an embodiment of the method as described herein, wherein the cancer is refractory and/or resistant to chemotherapy or radiation.

Other aspects and features of the present invention will become apparent to those of ordinary skill in the art upon review of the following description of specific embodiments of the invention in conjunction with the accompanying figures.

### BRIEF DESCRIPTION OF THE FIGURES

Figure 1 shows the potency of 5P12-RANTES at inhibiting signaling through human CCR5 in comparison with various known CCR5 inhibitors.
Figure 2 shows comparison of anti-HIV potency of 5P12-RANTES with that of AZT and MVC.
Figure 3 shows that 5P12-RANTES antagonizes neither CCR1 nor CCR3.
Figure 4 shows the efficacy of SP12-RANTES in an animal model of colorectal cancer. "QD" indicates daily dosing and "Q3D" indicates dosing every third day.

### DETAILED DESCRIPTION

The present inventors have surprisingly discovered that an N-terminally modified RANTES variant, namely, 5P12-RANTES (SEQ ID NO: 70), is much more potent than maraviroc (MVC) in inhibiting CCR5 signaling and/or HIV-1 replication. The present inventors have further demonstrated that, like MVC, 5P12-RANTES is selective for CCR5 over CCR1 and CCR3. Moreover, during the preclinical evaluation of 5P12-RANTES, the compound has been administered to cynomolgus macaques and rats as single dose intravenous infusion at a dose of 20 mg/kg without any adverse events. These findings indicate that this N-terminally modified RANTES variant and its structurally related analogs have enhanced efficacy compared to MVC which is known to be therapeutically effective against several types of cancer, with very little side effects. The present inventors have demonstrated the efficacy of 5P12-RANTES in an animal model of colorectal cancer.

Accordingly, the present invention relates to a CCR5 inhibitor for use in treating cancer by administering to a subject the CCR5 inhibitor, which is comprising an N-terminal portion and a C-terminal portion, wherein the N-terminal portion comprises the signature sequence QGP[P or L], i.e., the fourth position of the signature sequence may be either P or L, and the amino acid sequence of the C-terminal portion is at least 70% identical to SEQ ID NO: 1.

Without being limited by theory, it is believed that CCR5 inhibitors provided herein are capable of suppressing growth of cancer cells and/or reprogramming immunomodulatory leukocytes (e.g., inducing a phenotypic shift in "tumour-infiltrating macrophages from an immunosuppressive (M2) phenotype to an immunostimulatory (M1) phenotype). It is further believed that CCR5 inhibitors provided herein are not sensitive to tumor mutation load.

### CCR5 Inhibitors

As used herein, the term "CCR5 inhibitor" means a molecule or compound that inhibits one or more biological and/or pathological activities induced by CCR5. CCR5 inhibitors may prevent the binding of ligands or pathogens by achieving partial or complete occupation of the site or sites on CCR5 that they require for interaction (orthosteric inhibitors). Alternatively, CCR5 inhibitors may prevent the binding of ligands or pathogens by engaging other sites on CCR5 and inducing CCR5 to adopt a conformation or conformations that cannot be recognized by the ligands or pathogens (allosteric inhibitors). CCR5 inhibitors may inhibit the entire repertoire or only a subset of CCR5 intracellular signaling pathways. CCR5 inhibitors may also act by eliciting long-term intracellular sequestration of CCR5 so that it cannot be accessed by extracellular ligands or pathogens. CCR5 inhibitors may also act by specifically inhibiting or removing expression of the CCR5 gene. CCR5 inhibitors may also act by binding to one or more ligands or pathogens so that they are unable to bind to CCR5.

CCR5 inhibitors that may be used in the present invention are highly potent inhibitors of HIV entry into cells, i.e. have high anti-HIV potency, and are selective for CCR5 over the receptors CCR1 and CCR3; and may further have the ability to bind to CCR5 in its multiple cell surface conformations, and/or elicit only a low degree of pro-inflammatory signaling, and/or inhibit only a subset of the repertoire of CCR5 intracellular signaling pathways, and/or elicit the internalisation of CCR5 into the cell (receptor sequestration, down-regulation or down-modulation).

In the context of the present invention, the expressions "high anti-HIV potency", "high potency" or "highly potent" are used with regard to CCR5 inhibitors having an IC50 value, as measured by the cell fusion or HIV replication assay as described in the Materials and Methods section of US Patent No. 8,686,111, of 1000 pM (1 nM) or lower, for example, less than 900, 800, 700, 600, 500, 400, 300, 200, 150, 140, 130, 120, 110, 100, 90, 80, 70, 60, 50, 40, 30, or 20 pM. In some embodiments, CCR5 inhibitors that may be used in the methods of the present invention are more potent than MVC, for example, at least 2 times, 3 times, 4 times, 5 times, 6 times, 7 times, 8 times, 9 times, 10 times, 11 times, 12 times, 13 times, 14 times, 15 times, 16 times, 17 times, 18 times, 19 times, 20 times, 21 times, 22 times, 23 times, 24 times, 25 times, 26 times, 27 times, 28 times, 29 times, or 30 times, more potent than MVC as measured by the CCR5 signaling assay as described in Example 1 or the HIV replication assay as described in Example 2 of the present application.

In the context of the present invention, a CCR5 inhibitor that binds to CCR1 and/or CCR3 as well as CCR5 is still considered to have selectivity for CCR5 over CCR1 and/or CCR3 if it does not substantially activate CCR1 and/or CCR3. In some embodiments, CCR5 inhibitors that may be used in the methods of the present invention neither substantially bind nor substantially activate CCR1 and CCR3. In the context of the present invention, a CCR5 inhibitor is considered not to substantially bind CCR1 and/or CCR3 if the IC50 value of the CCR5 inhibitor with respect to CCR1 and/or CCR3 binding is greater than 50 nM, for example, greater than 60, 70, 80, 90, 100, 110, 120, 130, 140, 150, 200, 300, 400, 500, 600, 700, 800, 900 or 1000 nM, as measured using the CCR1 Discrimination Binding Assay and/or the CCR3 Discrimination Binding Assay as described in the Materials and Methods section of US Patent No. 8,686,111. Selectivity of CCR5 activation over CCR1 and/or CCR3 activation may be evaluated by means of the Calcium Flux Assay as described in the Materials and Methods section of US Patent No. 8,686,111. In the context of the present invention, a CCR5 inhibitor is considered not to substantially activate CCR1 and/or CCR3 if its signaling activity is less than 30%, for example, less than 26%, 22%, 20%, 18%, 16%, 14%, 12%, 10%, 8%, 6%, 4%, 2% or 1% of the Emax elicited on that receptor by native RANTES/CCL5, as measured by the Calcium Flux Assay as described in the Materials and Methods section of US Patent No. 8,686,111.

In the context of the present invention, a CCR5 inhibitor is considered to bind to CCR5 in both G protein-coupled and uncoupled conformations if its binding to CCR5 is unaffected by the presence or absence of a non-hydrolysable GTP analog such as GTPγS (guanosine 5'-O-(γ-thio)-triphosphate) or Gpp(NH)p (guanosine 5'-((β,γ-imido)triphosphate). The binding of a CCR5 inhibitor to CCR5 is considered unaffected by the presence or absence of a non-hydrolysable GTP analog if the difference in specifically bound radiolabelled inhibitor differs by less than 20% , for example, less than 18%, 16%, 14%, 12%, 10%, 8%, 6%, 5%, 4%, 3%, 2% or 1% in the presence or absence or 100 µM Gpp(NH)p, as measured by the radioligand binding assay as described in Figures 3D and 3E and in the Supporting Information of Colin P. et al. Proc Natl Acad Sci USA. 2013; 110(23):9475-9480.

In the context of the present invention, CCR5 inhibitors with "low signaling activity", i.e., their administration and/or binding to CCR5 causes only a low degree of pro-inflammatory signaling in target cells, lead to a signaling response of 30% or less of the maximum response (Emax) elicited by PSC-RANTES, when tested at a concentration of 300 nM in the Calcium Flux signaling assay as described in the Materials and Methods section of US Patent No. 8,686,111. For example, CCR5 inhibitors used in the methods of the present invention may have signaling activities, as measured in the Calcium Flux signaling assay, of less than 30%, 26%, 22%, 20%, 18%, 16%, 14%, 12%, 10%, 8%, 6%, 4%, 2% or 1% of the maximum response (Emax) elicited by PSC-RANTES. The sequence of PSC-RANTES is R¹-SSDTTPCCF A YIARPLPRAHIKEYFYTSGKCSNP A VVFVTRKNRQVCANPEKK WVREYINSLEMS, where R¹ = *N*^{α}-(*n*-nonanoyl)-*des*-Ser¹-[_{L}-thioprolyl², _{L}-cyclohexylglycyl³].

In the context of the present invention, CCR5 inhibitors with "high receptor sequestration activity", i.e. their administration and/or binding to CCR5 causes a high degree of receptor sequestration (said sequestration may be receptor internalisation, down-regulation or down-modulation), lead to the sequestration of at least 50% of the control level of surface CCR5 molecules, when tested in the CCR5 Surface Downmodulation/receptor sequestration assay as described in the Materials and Methods section of US Patent No. 8,686,111. For example, CCR5 inhibitors may have receptor sequestration activities of more than 50%, 55%, 60%, 65%, 70%, 80%, 85%, 90%, or 95% of the control level of surface CCR5.

In some embodiments, CCR5 inhibitors that may be used in the present invention are those disclosed in US Patent No. 8,686,111, namely, polypeptides that comprise an N-terminal portion and a C-terminal portion, wherein the N-terminal portion comprises the signature sequence QGP[P or L], i.e., the fourth position of the signature sequence may be either P or L, and the amino acid sequence of the C-terminal portion is at least 70% identical to SEQ ID NO: 1.

CCR5 inhibitors disclosed in US Patent No. 8,686,11 1may combine high anti-HIV potency with low signaling activity, or combine high anti-HIV potency with high receptor sequestration activity. In some embodiments, CCR5 inhibitors that may be used in the methods of the present invention combine high anti-HIV potency with both low signaling and high receptor sequestration activity. In some embodiments, CCR5 inhibitors that may be used in the methods of the present invention combine high anti-HIV potency with low signaling activity, but not high receptor sequestration activity.

In some embodiments, CCR5 inhibitors that may be used in the present invention are characterised by a combination of high levels of anti-HIV potency (IC50 levels, as measured by the Cell Fusion Assay as described in the Materials and Methods section of US Patent No. 8,686,111, of e.g., between 0.02 and 1 nM, or between 0.02 and 0.15 nM) with low levels of signaling activity (e.g., no more than 15%, no more than 10%, or no more than 5%, as measured by the Calcium Flux Assay as described in the Materials and Methods section of US Patent No. 8,686,111) and low levels of receptor sequestration activity (e.g., no more than 20%, or no more than 10%, as measured by the CCR5 Surface Downmodulation Assay as described in the Materials and Methods section of US Patent No. 8,686,111).

In some embodiments, CCR5 inhibitors that may be used in the present invention are characterised by a combination of high levels of anti-HIV potency (IC50 levels, as measured by the Cell Fusion Assay as described in the Materials and Methods section of US Patent No. 8,686,111, of e.g., between 0.02 and 1 nM, or between 0.02 and 0.15 nM) with high levels of signaling activity (e.g., at least 60%, or at least 85% as measured by the Calcium Flux Assay as described in the Materials and Methods section of US Patent No. 8,686,111) and high levels of receptor sequestration activity (e.g., at least 60%, or at least 70%, as measured by the CCR5 Surface Downmodulation Assay as described in the Materials and Methods section of US Patent No. 8,686,111).

In some embodiments, CCR5 inhibitors that may be used in the present invention are characterised by a combination of high levels of anti-HIV potency (IC50 levels, as measured by the Cell Fusion Assay as described in the Materials and Methods section of US Patent No. 8,686,111, of e.g., between 0.02 and 1 nM, or between 0.02 and 0.15 nM) with low levels of signaling activity (e.g., no more than 30%, or no more than 20%, no more than 15%, or no more than 10%, as measured by the Calcium Flux Assay as described in the Materials and Methods section of US Patent No. 8,686,111) and intermediate levels of receptor sequestration activity (e.g., between 20% and 50%, or between 30% and 50%, as measured by the CCR5 Surface Downmodulation Assay as described in the Materials and Methods section of US Patent No. 8,686,111).

In some embodiments, CCR5 inhibitors that may be used in the present invention are characterised by a combination of high levels of anti-HIV potency (IC50 levels, as measured by the Cell Fusion Assay as described in the Materials and Methods section of US Patent No. 8,686,111, of e.g., between 0.02 and 1 nM, or between 0.02 and 0.15 nM) with low levels of signaling activity (e.g., no more than 30%, or no more than 20%, no more than 15%, or no more than 10%, as measured by the Calcium Flux Assay as described in the Materials and Methods section of US Patent No. 8,686,111) and high levels of receptor sequestration activity (e.g., at least 50%, as measured by the CCR5 Surface Downmodulation Assay as described in the Materials and Methods section of US Patent No. 8,686,111).

In some embodiments, CCR5 inhibitors that may be used in the present invention are characterised by a combination of high levels of anti-HIV potency (IC50 levels, as measured by the Cell Fusion Assay as described in the Materials and Methods section of US Patent No. 8,686,111, of e.g., between 0.02 and 1 nM, or between 0.02 and 0.15 nM) with intermediate levels of signaling activity (e.g., between 30% and 50%, or between 30% and 45%, as measured by the Calcium Flux Assay as described in the Materials and Methods section of US Patent No. 8,686,111) and high levels of receptor sequestration activity (e.g., at least 50%, as measured by the CCR5 Surface Downmodulation Assay as described in the Materials and Methods section of US Patent No. 8,686,111).

In some embodiments, CCR5 inhibitors that may be used in the present invention are characterised by a combination of intermediate levels of anti-HIV potency (IC50 levels, as measured by the Cell Fusion Assay as described in the Materials and Methods section of US Patent No. 8,686,111, of e.g., between 0.02 and 1 nM, or between 0.02 and 0.15 nM) with intermediate levels of signaling activity (e.g., between 30% and 50%, or between 30% and 45% as measured by the Calcium Flux Assay as described in the Materials and Methods section of US Patent No. 8,686,111) and intermediate levels of receptor sequestration activity (e.g., between 20% and 50%, or between 30% and 50%, as measured by the CCR5 Surface Downmodulation Assay as described in the Materials and Methods section of US Patent No. 8,686,111).

In some embodiments, CCR5 inhibitors that may be used in the present invention comprise an N-terminal portion and a C-terminal portion, wherein the N-terminal portion comprises a signature sequence and the amino acid sequence of the C-terminal portion is at least 70% identical to SEQ ID NO: 1, wherein the signature sequence is: QGP[P or L][L or G or S or M][M or D or S or Q or G]; QGP[P or L][L or G][M or D or S]; QGP[P or L][L or G or S or M][M or D or S or Q or G]XX[Q or G or L or A or T or S]X, wherein X denotes any natural or modified amino acid; QGP[P or L][L or G][M or D or S]XX[Q or L]X, wherein X denotes any natural or modified amino acid; QGP[P or L] LM; QGPPG[D or S]; QGPPLM; or QGPPGD.

In some embodiments, CCR5 inhibitors that may be used in the present invention comprise an N-terminal portion and a C-terminal portion, wherein the N-terminal portion comprises a signature sequence and the amino acid sequence of the C-terminal portion is at least 70% identical to SEQ ID NO: 1, wherein the signature sequence is: QGP[P or L][L or M][M or Q][A or W or G or Q or N]X[Q or G or L][S or V or T or G], wherein X denotes any natural or modified amino acid; QGP[P or L][L or M][M or Q][A or W or G or Q or N][L or T or M or S or G or Q or R or Y][Q or G or L][S or V or T or G]; QGP[P or L]LM[A or W][L or T or M][Q or G][S or V or T or G]; or QGPPLM[A or W][L or T or M][Q or G][S or V or T or G].

In some embodiments, CCR5 inhibitors that may be used in the present invention comprise an N-terminal portion and a C-terminal portion, wherein the N-terminal portion comprises a signature sequence and the amino acid sequence of the C-terminal portion is at least 70% identical to SEQ ID NO: 1, wherein the signature sequence is: QGP[P or L][L or G or S][D or S or G or Q] XX[L or A or T or Q][W or A or V], wherein X denotes any natural or modified amino acid; QGP[P or L][L or G or S][D or S or G or Q][T or I or S or W or Q][V or L or A or S or G][L or A or T or Q][W or A or V]; QGPPG[D or S][T or I]VL[W or A]; or QGPPGD[T or I]VL[W or A].

In some embodiments, CCR5 inhibitors that may be used in the present invention comprise an N-terminal portion and a C-terminal portion, wherein the N-terminal portion comprises a signature sequence and the amino acid sequence of the C-terminal portion is at least 70% identical to SEQ ID NO: 1, wherein the signature sequence is: QGPP[G or L][M or Q]XX[Q or S][S or V], wherein X denotes any natural or modified amino acid; QGPP[G or L][M or Q] [S or G or W or A or T] [L or F or T or S or G or Y][Q or S][S or V]; or QGPPLM[S or G][L or For T]Q[S or V].

In some embodiments, CCR5 inhibitors that may be used in the present invention comprise an N-terminal portion and a C-terminal portion, wherein the N-terminal portion comprises a signature sequence and the amino acid sequence of the C-terminal portion is at least 70% identical to SEQ ID NO: 1, wherein the signature sequence is selected from the group QGPPLMALQS, QGPPLMWMQV, QGPPLMWLQV, QGPPLMWTQS, QGPPLMWLQT, QGPPLMWTQV, QGPPLMWMQS, QGPPLMATQS, QGPPLMWLQS, QGPPLMALQV, QGPPLMWLGG, QGPPLMWRGS, QGPLLMWLQV, QGPPLMQTTP, QGPPLSWLQV, QGPPLSWLQS, QGPPGQWSQV, QGPPMMAGLS, QGPPLSWQQS, QGPPGMWSQS, QGPPLQWRQS, QGPPLMGTQS, QGPPLMQLQV, QGPPLSWSQV, QGPPMSWSQS, QGPPLMNLQV, QGPPMSAYQV and QGPPMQGGLS.

In some embodiments, CCR5 inhibitors that may be used in the present invention comprise an N-terminal portion and a C-terminal portion, wherein the N-terminal portion comprises a signature sequence and the amino acid sequence of the C-terminal portion is at least 70% identical to SEQ ID NO: 1, wherein the signature sequence is selected from the group QGPPLMALQS, QGPPLMWMQV, QGPPLMWLQV, QGPPLMWTQS, QGPPLMWLQT, QGPPLMWTQV, QGPPLMWMQS, QGPPLMATQS, QGPPLMWLQS, QGPPLMALQV, QGPPLMWLGG, QGPPLMWRGS, QGPLLMWLQV, and QGPPLMQTTP.

In some embodiments, CCR5 inhibitors that may be used in the present invention comprise an N-terminal portion and a C-terminal portion, wherein the N-terminal portion comprises a signature sequence and the amino acid sequence of the C-terminal portion is at least 70% identical to SEQ ID NO: 1, wherein the signature sequence is selected from the group QGPPGDTVLW, QGPPGDIVLA, QGPPGSYDYS, QGPPGDGGSV, QGPLSGQSTP, QGPPGDWLQV, QGPPLMSLAV, QGPPLMSLTV, QGPLSGWAQV, QGPLSQSSQV, QGPLSSQSQV and QGPLGQQGQV.

In some embodiments, CCR5 inhibitors that may be used in the present invention comprise an N-terminal portion and a C-terminal portion, wherein the N-terminal portion comprises a signature sequence and the amino acid sequence of the C-terminal portion is at least 70% identical to SEQ ID NO: 1, wherein the signature sequence is selected from the group QGPPGDTVLW, QGPPGDIVLA, QGPPGSYDYS, QGPPGDGGSV, QGPLSGQSTP, and QGPPGDWLQV.

In some embodiments, CCR5 inhibitors that may be used in the present invention comprise an N-terminal portion and a C-terminal portion, wherein the N-terminal portion comprises a signature sequence and the amino acid sequence of the C-terminal portion is at least 70% identical to SEQ ID NO: 1, wherein the signature sequence is selected from the group QGPPLMSFQS, QGPPLMSTQS, QGPPLMSLQV, QGPPLMGLQV, QGPLSGWLQV, QGPPLQWFQV, QGPPLQWTQV, QGPPLMALSV, QGPPLMWSQV, QGPPGQWGQV, QGPPGSWSQV, QGPPLMSSQS, QGPPLMGLSV, QGPPLMTLQV and QGPPGQWYQS.

In some embodiments, CCR5 inhibitors that may be used in the present invention comprise an N-terminal portion and a C-terminal portion, wherein the N-terminal portion comprises a signature sequence and the amino acid sequence of the C-terminal portion is at least 70% identical to SEQ ID NO: 1, wherein the signature sequence is selected from the group QGPPLMSFQS, QGPPLMSTQS, QGPPLMSLQV, QGPPLMGLQV, and QGPLSGWLQV.

In some embodiments, CCR5 inhibitors that may be used in the present invention comprise an N-terminal portion and a C-terminal portion, wherein the N-terminal portion comprises a signature sequence and the amino acid sequence of the C-terminal portion is at least 70% identical to SEQ ID NO: 1, wherein the signature sequence is selected from the group QGPPLMSVLA, QGPPGSWSSV, QGPPLGSMGP, QGPPLQWMQA, QGPPLQWMQV, QGPPLMSTQV, QGPPLMSLSV, QGPPLMSLQS, QGPPLMSLQA, QGPPLMSVQS, QGPPLMSAQS, QGPPLMSGQS and QGPPLMSGQV.

In some embodiments, CCR5 inhibitors that may be used in the present invention comprise an N-terminal portion and a C-terminal portion, wherein the N-terminal portion comprises a signature sequence and the amino acid sequence of the C-terminal portion is at least 70% identical to SEQ ID NO: 1, wherein the signature sequence is selected from the group QGPPLMSVLA, QGPPGSWSSV, and QGPPLGSMGP.

In some embodiments, CCR5 inhibitors that may be used in the present invention comprise an N-terminal portion and a C-terminal portion, wherein the N-terminal portion comprises a signature sequence and the amino acid sequence of the C-terminal portion is at least 70% identical to SEQ ID NO: 1, wherein the signature sequence is selected from the signature sequences set out in Table 1:

**TABLE 1**

| SEQ ID NO | Signature Sequence |
|---|---|
| SEQ ID NO: 2 | QGPPLMALQS |
| SEQ ID NO: 3 | QGPPLMWMQV |
| SEQ ID NO: 4 | QGPPLMWLQV |
| SEQ ID NO: 5 | QGPPLMWTQS |
| SEQ ID NO: 6 | QGPPLMWLQT |
| SEQ ID NO: 7 | QGPPLMWTQV |
| SEQ ID NO: 8 | QGPPLMWMQS |
| SEQ ID NO: 9 | QGPPLMATQS |
| SEQ ID NO: 10 | QGPPLMWLQS |
| SEQ ID NO: 11 | QGPPLMALQV |
| SEQ ID NO: 12 | QGPPLMWLGG |
| SEQ ID NO: 13 | QGPPLMWRGS |
| SEQ ID NO: 14 | QGPLLMWLQV |
| SEQ ID NO: 15 | QGPPLMQTTP |
| SEQ ID NO: 16 | QGPPGDTVLW |
| SEQ ID NO: 17 | QGPPGDIVLA |
| SEQ ID NO: 18 | QGPPGSYDYS |
| SEQ ID NO: 19 | QGPPGDGGSV |
| SEQ ID NO: 20 | QGPLSGQSTP |
| SEQ ID NO: 21 | QGPPGDWLQV |
| SEQ ID NO: 22 | QGPPLMSFQS |
| SEQ ID NO: 23 | QGPPLMSTQS |
| SEQ ID NO: 24 | QGPPLMSLQV |
| SEQ ID NO: 25 | QGPPLMGLQV |
| SEQ ID NO: 26 | QGPLSGWLQV |
| SEQ ID NO: 27 | QGPPLMSVLA |
| SEQ ID NO: 28 | QGPPGSWSSV |
| SEQ ID NO: 29 | QGPPLGSMGP |
| SEQ ID NO: 30 | QGPPLSWLQV |
| SEQ ID NO: 31 | QGPPLSWLQS |
| SEQ ID NO: 32 | QGPPGQWSQV |
| SEQ ID NO: 33 | QGPPMMAGLS |
| SEQ ID NO: 34 | QGPPLSWQQS |
| SEQ ID NO: 35 | QGPPGMWSQS |
| SEQ ID NO: 36 | QGPPLQWRQS |
| SEQ ID NO: 37 | QGPPLMGTQS |
| SEQ ID NO: 38 | QGPPLMQLQV |
| SEQ ID NO: 39 | QGPPLSWSQV |
| SEQ ID NO: 40 | QGPPMSWSQS |
| SEQ ID NO: 41 | QGPPLMNLQV |
| SEQ ID NO: 42 | QGPPMSAYQV |
| SEQ ID NO: 43 | QGPPMQGGLS |
| SEQ ID NO: 44 | QGPPLMSLAV |
| SEQ ID NO: 45 | QGPPLMSLTV |
| SEQ ID NO: 46 | QGPLSGWAQV |
| SEQ ID NO: 47 | QGPLSQSSQV |
| SEQ ID NO: 48 | QGPLSSQSQV |
| SEQ ID NO: 49 | QGPLGQQGQV |
| SEQ ID NO: 50 | QGPPLQWFQV |
| SEQ ID NO: 51 | QGPPLQWTQV |
| SEQ ID NO: 52 | QGPPLMALSV |
| SEQ ID NO: 53 | QGPPLMWSQV |
| SEQ ID NO: 54 | QGPPGQWGQV |
| SEQ ID NO: 55 | QGPPGSWSQV |
| SEQ ID NO: 56 | QGPPLMSSQS |
| SEQ ID NO: 57 | QGPPLMGLSV |
| SEQ ID NO: 58 | QGPPLMTLQV |
| SEQ ID NO: 59 | QGPPGQWYQS |
| SEQ ID NO: 60 | QGPPLQWMQA |
| SEQ ID NO: 61 | QGPPLQWMQV |
| SEQ ID NO: 62 | QGPPLMSTQV |
| SEQ ID NO: 63 | QGPPLMSLSV |
| SEQ ID NO: 64 | QGPPLMSLQS |
| SEQ ID NO: 65 | QGPPLMSLQA |
| SEQ ID NO: 66 | QGPPLMSVQS |
| SEQ ID NO: 67 | QGPPLMSAQS |
| SEQ ID NO: 68 | QGPPLMSGQS |
| SEQ ID NO: 69 | QGPPLMSGQV |

In one embodiment, the CCR5 inhibitor used in the present invention comprises an N-terminal portion and a C-terminal portion, wherein the N-terminal portion comprises the signature sequence QGPPLMATQS and the amino acid sequence of the C-terminal portion is at least 70% identical to SEQ ID NO: 1.

In one embodiment, the CCR5 inhibitor used in the present invention comprises an N-terminal portion and a C-terminal portion, wherein the N-terminal portion comprises the signature sequence QGPPGDIVLA and the amino acid sequence of the C-terminal portion is at least 70% identical to SEQ ID NO: 1.

In one embodiment, the CCR5 inhibitor used in the present invention comprises an N-terminal portion and a C-terminal portion, wherein the N-terminal portion comprises the signature sequence QGPPLMSLQV and the amino acid sequence of the C-terminal portion is at least 70% identical to SEQ ID NO: 1.

In one embodiment, the CCR5 inhibitor used in the present invention comprises an N-terminal portion and a C-terminal portion, wherein the N-terminal portion consists of the signature sequence QGPPLMATQS and the amino acid sequence of the C-terminal portion is SEQ ID NO: 1.

In one embodiment, the CCR5 inhibitor used in the present invention comprises an N-terminal portion and a C-terminal portion, wherein the N-terminal portion consists of the signature sequence QGPPGDIVLA and the amino acid sequence of the C-terminal portion is SEQ ID NO: 1.

In one embodiment, the CCR5 inhibitor used in the present invention comprises an N-terminal portion and a C-terminal portion, wherein the N-terminal portion consists of the signature sequence QGPPLMSLQV and the amino acid sequence of the C-terminal portion is SEQ ID NO: 1.

In one embodiment, the CCR5 inhibitor used in the present invention comprises the amino acid sequence of SEQ ID NO: 70.

In one embodiment, the CCR5 inhibitor used in the present invention comprises the amino acid sequence of SEQ ID NO: 71.

In one embodiment, the CCR5 inhibitor used in the present invention comprises the amino acid sequence of SEQ ID NO: 72.

The N-terminal portion of the CCR5 inhibitors described in paragraphs [0044] to [0067] may consist of no more than 15, 14, 13, 12, 11, or 10 amino acids. In one embodiment, said N-terminal portion consists of 10 amino acids.

In the CCR5 inhibitors described in paragraphs [0044] to [0067], the N-terminus of the C-terminal portion may adjoin directly to the C-terminus of the N-terminal portion, i.e. the N-terminal portion and the C-terminal portion are directly adjoined.

The C-terminal portion of the CCR5 inhibitors described in paragraphs [0044] to [0067] may have more than 70%, 75%, 80%, 85%, 90%, 95%, 98%, 99%, 99.9% or 100% sequence identity to SEQ ID NO: 1.

The term "sequence identity," as used herein, has the standard meaning in the art. As is known in the art, a number of different programs can be used to identify whether a polynucleotide or polypeptide has sequence identity or similarity to a known sequence. Sequence identity or similarity may be determined using standard techniques known in the art, including, but not limited to, the local sequence identity algorithm of Smith & Waterman, Adv. Appl. Math. 2:482 (1981), by the sequence identity alignment algorithm of Needleman & Wunsch, J. Mol. Biol. 48:443 (1970), by the search for similarity method of Pearson & Lipman, Proc. Natl. Acad. Sci. USA 85:2444 (1988), by computerized implementations of these algorithms (GAP, BESTFIT, FASTA, and TFASTA in the Wisconsin Genetics Software Package, Genetics Computer Group, 575 Science Drive, Madison, WI), the Best Fit sequence program described by Devereux et al., Nucl. Acid Res. 12:387 (1984), preferably using the default settings, or by inspection.

The signature sequence of the CCR5 inhibitors described in paragraphs [0044] to [0067] may be located near the N-terminus of the polypeptide. For example, the signature sequence may be located such that the beginning of the signature sequence lies within 15, 12, 10, 8, 6, 5, 4, 3, 2, or 1 residue of the N-terminus of the polypeptide. Herein, the expression "the beginning of the signature sequence" refers to the N-terminus of the signature sequence. The signature sequence may also be located at the extreme N-terminus of the polypeptide, i.e., the N-termini of the polypeptide as a whole and the signature sequence may coincide.

In some embodiments, nucleic acids encoding the polypeptides provided herein may be used in the present invention. The skilled person would know how to design or identify nucleic acids encoding the polypeptides, according to the genetic code. Such a nucleic acid may comprise one or more segments encoding one or more polypeptides provided herein. Such a nucleic acid may be RNA or DNA. Such a nucleic acid may be a vector, i.e. nucleic acids encoding the polypeptides provided herein may be incorporated within a vector.

In some embodiments, nucleic acids encoding the polypeptides provided herein may be incorporated into a virus. Thus, a virus that contains, within its genome, one or more segments encoding one or more polypeptides provided herein, may be used in the methods of the present invention.

As used herein, the terms "protein", "peptide" or "polypeptide" are used interchangeably and refer to amino acid polymers of any length. The polymer may be linear or branched, it may comprise modified amino acids, and it may be interrupted by non-amino acids. The terms also encompass an amino acid polymer that has been modified naturally or by intervention; for example, disulfide bond formation, glycosylation, lipidation, acetylation, phosphorylation, or any other manipulation or modification, such as conjugation with a labelling component. Also included within the definition are, for example, polypeptides containing one or more analogues of an amino acid (including, for example, unnatural amino acids, etc.), as well as other modifications known in the art. Polypeptides can occur as single chains or associated chains. Polypeptides of the invention can be naturally or non-naturally glycosylated (i.e. the polypeptide has a glycosylation pattern that differs from the glycosylation pattern found in the corresponding naturally occurring polypeptide).

### Pharmaceutical Compositions and Dosage Forms

The present invention provides compositions, for example, pharmaceutical compositions, comprising CCR5 inhibitors for use according to the present invention. As used herein, the terms "pharmaceutical composition" and "composition" may be used interchangeably, as the context requires. A pharmaceutical composition as disclosed herein may be administered to a subject in a therapeutically effective amount. As used herein, a "therapeutically effective amount" means an amount of the composition or therapeutic agent effective to provide a therapeutic, prophylactic or diagnostic benefit to a subject. In some embodiments, a therapeutically effective amount of the composition is an amount capable of inducing a clinical response in a subject in the treatment of a particular disease or disorder. Determination of a therapeutically effective amount of the composition is well within the capability of those skilled in the art, especially in light of the disclosure provided herein. The therapeutically effective amount may vary according to a variety of factors such as the subject's condition, weight, sex and age.

Pharmaceutical compositions provided herein may further comprise a pharmaceutically acceptable carrier, excipient, and/or stabilizer (Remington: The Science and practice of Pharmacy 20th Ed., 2000, Lippincott Williams and Wilkins, Ed. K. E. Hoover). Acceptable carriers, excipients, or stabilizers are nontoxic to recipients at the dosages and concentrations, and may comprise buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrans; chelating agents such as EDTA; sugars such as sucrose, mannitol, trehalose or sorbitol; salt-forming counter-ions such as sodium; metal complexes (e.g., Zn-protein complexes); and/or non-ionic surfactants such as TWEEN^{™}, PLURONICS^{™} or polyethylene glycol (PEG).

In some embodiments, pharmaceutical compositions provided herein may comprise more than one CCR5 inhibitor as described herein, e.g., two or more CCR5 inhibitors. In some embodiments, pharmaceutical compositions provided herein may comprise a second therapeutic agent that is known to have a therapeutic effect against cancer. As used herein, the term "therapeutic agent" is any molecule, substance or compound that is capable of providing a therapeutic activity, response or effect in the treatment or prevention of a disease, disorder or condition, including diagnostic and prophylactic agents.

Pharmaceutical compositions provided herein may be prepared in various pharmaceutical dosage forms, such as an instant release, controlled release, sustained release, or target drug-delivery system. Commonly used dosage forms include, for example, solutions and suspensions, (micro-) emulsions, ointments, gels, creams, pastes, foams, suppositories, ovules, implants, patches, liposomes, tablets, dragees, lozenges, soft or hard shell capsules, amorphous or crystalline powders, effervescent powders or tablets, aerosols, and lyophilized formulations. Depending on the route of administration used, special devices may be required for application or administration of a dosage form, such as syringes and needles, inhalers, pumps, injection pens, applicators, special flasks, or other devices for administration, which may also be implanted within a body.

Pharmaceutical dosage forms provided herein may be manufactured by any of the methods well-known in the art, such as, for example, by conventional mixing, sieving, dissolving, melting, granulating, dragee-making, tabletting, suspending, extruding, spray-drying, levigating, emulsifying, (nano/micro-) encapsulating, entrapping, or lyophilization processes.

For the present invention, CCR5 inhibitors, pharmaceutical compositions or dosage forms provided herein may be administered to a subject by conventional techniques, such as intravenously (as a bolus or by continuous infusion over a period of time), intramuscularly, transmucosally, intraperitoneally, intracerebrally, subcutaneously, intra-articularly, intrasynovially, intrathecally, nasally, orally, topically, or by inhalation. Other suitable administration routes may include intra-lesional or peri-lesional routes.

For intravenous injection, for example, pharmaceutical compositions provided herein may be formulated in aqueous solution, if necessary using physiologically compatible buffers, including, for example, phosphate, histidine, or citrate for adjustment of the formulation pH, and a tonicity agent, such as, for example, sodium chloride or dextrose. For transmucosal or nasal administration, semisolid, liquid formulations, or patches may be preferred, possibly containing penetration enhancers. Such penetrants are generally known in the art. For oral administration, pharmaceutical compositions provided herein may be formulated in liquid or solid dosage forms and optionally as instant or controlled/sustained release formulations. Suitable dosage forms for oral ingestion by a subject include tablets, capsules, pills, dragees, hard and soft shell capsules, liquids, gels, syrups, slurries, suspensions, and emulsions.

Solid oral dosage forms can be obtained using excipients, which may include inert diluents, fillers, disintegrants, binders (dry and wet), dissolution retardants, lubricants, glidants, antiadherants, cationic exchange resins, wetting agents, antioxidants, preservatives, colouring, sweetening and flavouring agents. These excipients can be of synthetic or natural source. Examples of such excipients include cellulose derivatives, citric acid, dicalcium phosphate, gelatine, magnesium carbonate, magnesium/sodium lauryl sulfate, mannitol, polyethylene glycol, polyvinyl pyrrolidone, silicates, silicon dioxide, sodium benzoate, sorbitol, starches, stearic acid or a salt thereof, sugars (i.e. dextrose, sucrose, lactose, etc.), talc, tragacanth mucilage, vegetable oils (hydrogenated), and waxes. Ethanol and water may serve as granulation aides. In certain instances, coating of tablets with, for example, a taste-masking film, a stomach acid resistant film, or a release-retarding film is desirable. Natural and synthetic polymers, in combination with colorants, sugars, and organic solvents or water, are often used to coat tablets, resulting in dragees. When a capsule is preferred over a tablet, the drug powder, suspension, or solution thereof can be delivered in a compatible hard or soft shell capsule.

Suitable inert diluents include sodium and calcium carbonate, sodium and calcium phosphate and lactose. Corn starch and alginic acid are suitable disintegrating agents. Binding agents may include starch and gelatine. The lubricating agent, if present, will generally be magnesium stearate, stearic acid or talc. If desired, the tablets may be coated with a material such as glyceryl monostearate or glyceryl distearate, to delay absorption in the gastrointestinal tract.

Capsules for oral use include hard gelatine capsules in which an active ingredient is mixed with a solid diluent and soft gelatine capsules wherein the active ingredient is mixed with water or an oil such as peanut oil, liquid paraffin or olive oil.

In some embodiments, CCR5 inhibitors provided herein may be administered topically, via the skin or mucous membrane, such as through a skin patch, a semi-solid or a liquid formulation, for example a gel, a (micro-) emulsion, an ointment, a solution, a (nano/micro)-suspension, or a foam. The penetration of an active ingredient into the skin or mucous membrane and underlying tissues of a subject can be regulated, for example, using penetration enhancers; the appropriate choice and combination of lipophilic, hydrophilic, and amphiphilic excipients, including water, organic solvents, waxes, oils, synthetic and natural polymers, surfactants, emulsifiers; by pH adjustment; and use of complexing agents.

In some embodiments, CCR5 inhibitors provided herein may be administered by inhalation, or to the nose, in the form of a solution, suspension, emulsion, or semisolid aerosol from pressurized packs, or a nebuliser, usually with the use of a propellant, e.g., halogenated carbons derived from methane and ethane, carbon dioxide, or any other suitable gas. For topical aerosols, hydrocarbons like butane, isobutene, and pentane are useful. In the case of a pressurized aerosol, the appropriate dosage unit may be determined by providing a valve to deliver a metered amount. Capsules and cartridges of, for example, gelatin, for use in an inhaler or insufflator, may be formulated. These typically contain a powder mix of an active ingredient and a suitable powder base such as lactose or starch.

Compositions formulated for parenteral administration by injection are usually sterile and, can be presented in unit dosage forms, e.g., in ampoules, syringes, injection pens, or in multi-dose containers, the latter usually containing a preservative. Pharmaceutical compositions suitable for parenteral administration may take such forms as suspensions, solutions, or emulsions in oily or aqueous vehicles, and may contain formulatory agents, such as buffers, tonicity agents, viscosity enhancing agents, surfactants, suspending and dispersing agents, antioxidants, biocompatible polymers, chelating agents, and preservatives. Depending on the injection site, the vehicle may contain water, a synthetic or vegetable oil, and/or organic co-solvents. In certain instances, such as with a lyophilized product or a concentrate, the parenteral formulation would be reconstituted or diluted prior to administration. Depot formulations, providing controlled or sustained release of an active agent, may include injectable suspensions of nano/micro particles or nano/micro or non-micronized crystals. Polymers such as poly(lactic acid), poly(glycolic acid), or copolymers thereof, can serve as controlled/sustained release matrices, in addition to others well known in the art. Other depot delivery systems may be presented in form of implants and pumps requiring incision.

Suitable carriers for intravenous injection of CCR5 inhibitors provided herein are well-known in the art and include water-based solutions containing a base, such as, for example, sodium hydroxide, to form an ionized agent, sucrose or sodium chloride as a tonicity agent. A water-based solution may comprise a buffer containing phosphate or histidine. Co-solvents, such as polyethylene glycols, may be added. These water-based systems are effective at dissolving agents and produce low toxicity upon systemic administration. The proportions of the components of a solution system may be varied considerably, without destroying solubility and toxicity characteristics. Furthermore, the identity of the components may be varied. For example, low-toxicity surfactants, such as polysorbates or poloxamers, may be used, as can polyethylene glycol or other co-solvents, biocompatible polymers such as polyvinyl pyrrolidone may be added, and other sugars and polyols may substitute for dextrose.

### Treatment Indications

CCR5 inhibitors, pharmaceutical compositions and dosage forms provided herein may find application in any instance in which it is desired to administer a therapeutic agent to a subject for treating or preventing cancer. As used herein, an "individual" or a "subject" is a mammal, for example, a human. Mammals also include, but are not limited to, farm animals, sport animals, pets, primates, and horses.

"Treating" or "treatment of", or "preventing" or "prevention of", as used herein, refers to an approach for obtaining beneficial or desired results. Beneficial or desired results can include, but are not limited to, alleviation or amelioration of one or more symptoms or conditions, diminishment of extent of disease, stabilisation of the state of disease, prevention of development of disease, prevention of spread of disease, delay or slowing of disease progression (e.g. suppression), delay or slowing of disease onset, conferring protective immunity against a disease-causing agent and amelioration or palliation of the disease state. "Treating" or "preventing" can also mean prolonging survival of a patient beyond that expected in the absence of treatment and can also mean inhibiting the progression of disease temporarily or preventing the occurrence of disease, such as by preventing infection in a subject.

"Treating" may be distinguished from "preventing" in that "treating" typically occurs in a subject who already has a disease or disorder, or is known to have already been exposed to an infectious agent, whereas "preventing" typically occurs in a subject who does not have a disease or disorder, or is not known to have been exposed to an infectious agent. As will be appreciated, there may be overlap in treatment and prevention. For example, it is possible to be "treating" a disease in a subject, while at same time "preventing" symptoms or progression of the disease.

As used herein, the term "cancer" refers to a condition in which cells proliferate at an abnormally high and poorly controlled or uncontrolled rate, the rate exceeding and uncoordinated with that of the surrounding normal tissues. Cancer may be primary or metastatic. Cancer can spread from where it started to another part of the body. The original cancer is called the primary tumor. The cancer in another part of the body is called metastatic cancer.

Cancer that may be treated by the method of the present invention may be refractory or resistant to conventional treatments such as chemotherapy or radiation. The term "refractory or resistant" refers to a circumstance where cancer does not respond to treatment. The cancer may be resistant at the beginning of treatment or it may become resistant during treatment.

Specific examples of cancer include, but are not limited to, cancers of the skin, such as melanoma and basal cell carcinoma; lymph node; breast; cervix; uterus; gastrointestinal tract, such as stomach (gastric) and gastrointestinal stromal tumors; lung; ovary; prostate; colon; rectum; mouth; brain; head and neck; throat; thyroid; testes; kidney; pancreas; bone; spleen; liver; bladder; larynx; nasal passages; AIDS-related cancers; sarcomas, such as soft tissue sarcoma, liposarcoma, and osteosarcoma; and cancers of the blood and bone marrow, such as multiple myeloma, myeloproliferative disorders, and acute and chronic leukemias, for example, lymphoblastic, myelogenous, lymphocytic, and myelocytic leukemias.

Other specific cancers include, but are not limited to, advanced malignancy, amyloidosis, neuroblastoma, meningioma, hemangiopericytoma, multiple brain metastases, glioblastoma multiforms, glioblastoma, brain stem glioma, poor prognosis malignant brain tumor, malignant glioma, recurrent malignant glioma, anaplastic astrocytoma, anaplastic oligodendroglioma, neuroendocrine tumor, rectal adenocarcinoma, Dukes C & D colorectal cancer, unresectable colorectal carcinoma, metastatic hepatocellular carcinoma, Kaposi's sarcoma, karotype acute myeloblastic leukemia, chronic lymphocytic leukemia (CLL), Hodgkin's lymphoma, non-Hodgkin's lymphoma, peripheral T-cell lymphoma (including anaplastic large cell lymphoma, angioimmunoblastic T-cell lymphoma, and cutaneous T-cell lymphoma), cutaneous B-cell lymphoma, diffuse large B-cell lymphoma, follicular lymphoma, mantle cell lymphoma, small lymphocytic lymphoma, Castleman's disease, metastatic melanoma (localized melanoma, including, but not limited to, ocular melanoma), malignant mesothelioma, malignant pleural effusion mesothelioma syndrome, peritoneal carcinoma, papillary serous carcinoma, gynecologic sarcoma, soft tissue sarcoma, scleroderma, cutaneous vasculitis, Langerhans cell histiocytosis, leiomyosarcoma, fibrodysplasia ossificans progressive, hormone refractory prostate cancer, resected high-risk soft tissue sarcoma, unrescectabie hepatocellular carcinoma, Waldenstrom's macroglobulinemia, smoldering myeloma, indolent myeloma, fallopian tube cancer, androgen independent prostate cancer, androgen dependent stage IV non-metastatic prostate cancer, hormone-insensitive prostate cancer, chemotherapy-insensitive prostate cancer, papillary thyroid carcinoma, follicular thyroid carcinoma, medullary thyroid carcinoma, and leiomyoma.

Cancer that may be treated by the present invention may be characterized by tumor cells that express CCR5 and/or by solid tumors infiltrated by leukocytes that express CCR5.

In a specific embodiment, the cancer is colorectal cancer. Histologic types of colon cancer include the following: adenocarcinoma (most colon cancers) which includes mucinous (colloid) adenocarcinoma and signet ring adenocarcinoma; scirrhous tumors; and neuroendocrine (tumors with neuroendocrine differentiation typically have a poorer prognosis than pure adenocarcinoma variants). Colorectal cancer treated by the methods of the present invention may be metastatic, refractory or resistant to chemotherapy or radiation, or both.

In a specific embodiment, the cancer is breast cancer. Of all women with breast cancer, 5% to 10% may have a germline mutation of the genes *BRCA1* and *BRCA2.* The estimated lifetime risk of developing breast cancer for women with *BRCA1* and *BRCA2* mutations is 40% to 85%. The use of molecular profiling in breast cancer includes the following: estrogen receptor (ER) and progesterone receptor (PR) status testing; HER2/neu receptor status testing; and gene profile testing by microarray assay or reverse transcription-polymerase chain reaction (e.g., MammaPrint, Oncotype DX). On the basis of ER, PR, and HER2/neu results, breast cancer is classified as one of the following types: hormone receptor positive; HER2/neu positive; and triple negative (ER, PR, and HER2/neu negative). ER, PR, and HER2 status are important in determining prognosis and in predicting response to endocrine and HER2-directed therapy. For example, hormone receptor (ER and/or PR)-positive patients will receive hormone therapy; HER2 overexpression is an indication for using adjuvant trastuzumab, usually in combination with chemotherapy; and when neither HER2 overexpression nor hormone receptors are present (i.e., triple-negative breast cancer), adjuvant therapy relies on chemotherapeutic regimens, which may be combined with investigational targeted approaches. Adjuvant treatment options may include the following: tamoxifen; aromatase inhibitor (AI) therapy; ovarian function suppression; and chemotherapy. Breast cancer treated by the methods of the present invention may be metastatic (for example, breast cancer may metastasize to the lung, liver, bone or brain), refractory or resistant to chemotherapy or radiation, or both.

In a specific embodiment, the cancer is lung cancer. The two main types of lung cancer are small cell lung cancer (SCLC) and non-SCLC (NSCLC); NSCLC accounts for approximately 85% of all cases of lung cancer. SCLC includes small cell carcinoma and combined small cell carcinoma, i.e., SCLC combined with neoplastic squamous and/or glandular components. The most common types of NSCLC are squamous cell carcinoma, large cell carcinoma, and adenocarcinoma. Lung cancer treated by the methods of the present invention may be metastatic, refractory or resistant to chemotherapy or radiation, or both. Metastatic sites in SCLC include liver, bone, brain and lung. Metastatic sites in NSCLC include bone, lung, brain, liver and adrenal glands.

In a specific embodiment, the cancer is prostate cancer. More than 95% of primary prostate cancers are adenocarcinomas. Prostate adenocarcinomas are frequently multifocal and heterogeneous in patterns of differentiation. Prostatic intraepithelial neoplasia ([PIN] noninvasive atypical epithelial cells within benign appearing acini) is often present in association with prostatic adenocarcinoma. PIN is subdivided into low grade and high grade. The high-grade form may be a precursor for adenocarcinoma. Studies have also estimated that within five years of diagnosis, 10-20% of men with prostate cancer will develop Castration-Resistant Prostate Cancer (CRPC), which further significantly decreased survival rate. Prostate cancer treated by the methods of the present invention may be metastatic, refractory or resistant to chemotherapy or radiation, or both.

In a specific embodiment, the cancer is ovarian cancer. Ovarian cancer includes ovarian epithelial cancer, fallopian tube cancer (FTC), and primary peritoneal cancer (PPC). Ovarian epithelial, fallopian tube, and primary peritoneal cancer can be histologically classified into serous cystomas, mucinous cystomas, endometrioid tumors (similar to adenocarcinomas in the endometrium), and clear cell (mesonephroid) tumors. Ovarian cancer treated by the methods of the present invention may be metastatic, refractory or resistant to chemotherapy or radiation, or both.

In a specific embodiment, the cancer is pancreatic cancer. Pancreatic cancer treated by the methods of the present invention may be metastatic, refractory or resistant to chemotherapy or radiation, or both.

In a specific embodiment, the cancer is esophageal cancer. Two histologic types account for the majority of malignant esophageal neoplasms: adenocarcinoma and squamous cell carcinoma. Adenocarcinomas typically start in the lower esophagus and squamous cell carcinoma can develop throughout the esophagus. Esophageal cancer treated by the methods of the present invention may be metastatic, refractory or resistant to chemotherapy or radiation, or both.

In a specific embodiment, the cancer is gastric cancer. There are two major types of gastric adenocarcinoma, intestinal and diffuse. Intestinal adenocarcinomas are well differentiated, and the cells tend to arrange themselves in tubular or glandular structures. The terms tubular, papillary, and mucinous are assigned to the various types of intestinal adenocarcinomas. Rarely, adenosquamous cancers can occur. Diffuse adenocarcinomas are undifferentiated or poorly differentiated, and they lack a gland formation. Clinically, diffuse adenocarcinomas can give rise to infiltration of the gastric wall (i.e., linitis plastica). Some tumors can have mixed features of intestinal and diffuse types. Gastric cancer treated by the methods of the present invention may be metastatic, refractory or resistant to chemotherapy or radiation, or both.

In a specific embodiment, the cancer is leukemia. Leukemia can be classified as acute leukemia or chronic leukemia. Leukemia can also be classified by the type of blood cell affected, namely lymphoblastic or lymphocytic leukemia, or myeloid or myelogenous leukemia. Leukemia treated by the methods of the present invention may be acute lymphoblastic leukemia (ALL). ALL is an aggressive type of leukemia characterized by the presence of too many lymphoblasts or lymphocytes in the bone marrow and peripheral blood. Leukemia treated by the methods of the present invention may be metastatic, refractory or resistant to chemotherapy or radiation, or both. It can spread to the lymph nodes, spleen, liver, central nervous system (CNS), and other organs.

In a specific embodiment, the cancer is liver cancer. Malignant primary tumors of the liver consist of two major cell types, which are hepatocellular (90% of cases) and cholangiocarcinoma. Histologic classification of liver tumors includes hepatocellular carcinoma (HCC), fibrolamellar variant of HCC, cholangiocarcinoma (intrahepatic bile duct carcinoma), mixed hepatocellular cholangiocarcinoma, undifferentiated tumours, and hepatoblastoma. Liver cancer treated by the methods of the present invention may be metastatic, refractory or resistant to chemotherapy or radiation, or both. Sites of metastasis of primary liver cancer include the lung, portal vein, and portal lymph nodes.

In some embodiments, treatment of cancer by the present invention may result in inhibition of cancer cell growth or proliferation. In some embodiments, treatment of cancer by the present invention may result in inhibition of cancer cell migration. In some embodiments, treatment of cancer by the present invention may result in inhibition of angiogenesis. In some embodiments, treatment of cancer by the present invention may result in reprogramming of immunomodulatory leukocytes.

According to some embodiments, a CCR5 inhibitor, pharmaceutical composition or dosage form provided herein is administered before the appearance of symptoms of cancer. According to some embodiments, the subject may be treated chronically for preventing metastasis and the damage caused therefrom. According to some embodiments, cancer is treated in patients that are resistant and/or not sensitive to conventional treatments, such as chemotherapy or radiation. According to some embodiments, the symptoms of cancer are treated by a CCR5 inhibitor, pharmaceutical composition or dosage form provided herein.

CCR5 inhibitors, pharmaceutical compositions and dosage forms provided herein may be administered by any means that achieve their intended purpose. For example, administration may be by carried out orally, sublingually, buccally, topically, rectally, via inhalation, transdermally, subcutaneously, intravenously, intra-arterially or intramuscularly, via intracardiac administration, intraosseously, intradermally, intraperitoneally, intracerebrally, transmucosally, vaginally, intravitreally, epicutaneously, intra-articularly, intravesically, intrathecally, peri-articularly or locally. The dosage administered will be dependent upon the age, health, and weight of the subject, kind of concurrent treatment, if any, frequency of treatment, and the nature of the effect desired.

According to some embodiments, a CCR5 inhibitor provided herein may be administered orally, possibly in the form of tablets, capsules, powders, troches, soft gelatin capsules, syrup, liquid suspension or lozenges. According to some embodiments, a CCR5 inhibitor provided herein may be administered in the form of a parenteral formulation, such as for subcutaneous intramuscular or intravenous administration. According to further embodiments, a CCR5 inhibitor provided herein may be administered by any appropriate nasal administration, pulmonary administration, topically or any appropriate dermatological administration.

According to some embodiments, a CCR5 inhibitor provided herein may be administered together with any appropriate non-toxic pharmaceutical carrier. The carrier may be a gas, solid or liquid. According to some embodiments, the carrier is selected from saline solution, water, any appropriate emulsion or dispersion or any combination thereof.

According to some embodiments, a CCR5 inhibitor provided herein may be administered together with any ingredients appropriate for modifying the release of the CCR5 inhibitor from the dosage form comprising the CCR5 inhibitor. For example, time delaying agents, such as enteric coated gelatin capsules, may be used.

According to some embodiments, in methods of treating cancer, a CCR5 inhibitor, pharmaceutical composition or dosage form provided herein may be administered together with any second active agents that are known to have therapeutic efficacy against cancer. Second active agents can be large molecules (e.g., proteins) or small molecules (e.g., synthetic inorganic, organometallic, or organic molecules).

Examples of large molecule active agents include, but are not limited to, hematopoietic growth factors, cytokines, and monoclonal and polyclonal antibodies. Specific examples of the active agents are anti-CD40 monoclonal antibodies (such as, for example, SGN-40); checkpoint inhibitors (such as, for example, anti-CTLA4 antibodies such as ipilimumab; anti-PD-1 antibodies such as nivolumab and pembrolizumab; and anti-PD-L-1 antibodies, such as atezolizumab, avelumab, and durvalumab); histone deacetylase inhibitors (such as, for example, SAHA and LAQ 824); heat-shock protein-90 inhibitors (such as, for example, 17-AAG); insulin-like growth factor-1 receptor kinase inhibitors; vascular endothelial growth factor receptor kinase inhibitors (such as, for example, PTK787); insulin growth factor receptor inhibitors; lysophosphatidic acid acyltransferase inhibitors; IkB kinase inhibitors; p38MAPK inhibitors; EGFR inhibitors (such as, for example, gefitinib and erlotinib HCL); HER-2 antibodies (such as, for example, trastuzumab (Herceptin^{®}) and pertuzumab (Omnitarg^{™})); VEGFR antibodies (such as, for example, bevacizumab (Avastin^{™})); VEGFR inhibitors (such as, for example, flk-1 specific kinase inhibitors, SU5416 and ptk787/zk222584); PI3K inhibitors (such as, for example, wortmannin); C-Met inhibitors (such as, for example, PHA 665752); monoclonal antibodies (such as, for example, rituximab (Rituxan^{®}), tositumomab (Bexxar^{®}), edrecolomab (Panorex^{®}) and G250); and anti-TNF-α antibodies. Examples of small molecule active agents include, but are not limited to, anticancer agents and antibiotics (e.g., clarithromycin).

Specific second active compounds that can be combined with CCR5 inhibitors provided herein vary depending on the specific indication to be treated, prevented or managed.

For instance, second active agents include, but are not limited to: semaxanib; cyclosporin; etanercept; doxycycline; bortezomib; lapatinib; acivicin; aclarubicin; acodazole hydrochloride; acronine; adozelesin; aldesleukin; altretamine; ambomycin; ametantrone acetate; amsacrine; anastrozole; anthramycin; asparaginase; asperlin; azacitidine; azetepa; azotomycin; batimastat; benzodepa; bicalutamide; bisantrene hydrochloride; bisnafide dimesylate; bizelesin; bleomycin sulfate; brequinar sodium; bropirimine; busulfan; cactinomycin; calusterone; caracemide; carbetimer; carboplatin; carmustine; carubicin hydrochloride; carzelesin; cedefingol; celecoxib; chlorambucil; cirolemycin; cisplatin; cladribine; crisnatol mesylate; cyclophosphamide; cytarabine; dacarbazine; dactinomycin; daunorubicin hydrochloride; decitabine; dexormaplatin; dezaguanine; dezaguanine mesylate; diaziquone; docetaxel; doxorubicin; doxorubicin hydrochloride; droloxifene; droloxifene citrate; dromostanolone propionate; duazomycin; edatrexate; eflomithine hydrochloride; elsamitrucin; enloplatin; enpromate; epipropidine; epirubicin hydrochloride; erbulozole; esorubicin hydrochloride; estramustine; estramustine phosphate sodium; etanidazole; etoposide; etoposide phosphate; etoprine; fadrozole hydrochloride; fazarabine; fenretinide; floxuridine; fludarabine phosphate; fluorouracil; flurocitabine; fosquidone; fostriecin sodium; gemcitabine; gemcitabine hydrochloride; hydroxyurea; idarubicin hydrochloride; ifosfamide; ilmofosine; iproplatin; irinotecan; irinotecan hydrochloride; lanreotide acetate; letrozole; leuprolide acetate; liarozole hydrochloride; lometrexol sodium; lomustine; losoxantrone hydrochloride; masoprocol; maytansine; mechlorethamine hydrochloride; megestrol acetate; melengestrol acetate; melphalan; menogaril; mercaptopurine; methotrexate; methotrexate sodium; metoprine; meturedepa; mitindomide; mitocarcin; mitocromin; mitogillin; mitomalcin; mitomycin; mitosper; mitotane; mitoxantrone hydrochloride; mycophenolic acid; nocodazole; nogalamycin; ormaplatin; oxisuran; paclitaxel; pegaspargase; peliomycin; pentamustine; peplomycin sulfate; perfosfamide; pipobroman; piposulfan; piroxantrone hydrochloride; plicamycin; plomestane; porfimer sodium; porfiromycin; prednimustine; procarbazine hydrochloride; puromycin; puromycin hydrochloride; pyrazofurin; riboprine; safingol; safingol hydrochloride; semustine; simtrazene; sparfosate sodium; sparsomycin; spirogermanium hydrochloride; spiromustine; spiroplatin; streptonigrin; streptozocin; sulofenur; talisomycin; tecogalan sodium; taxotere; tegafur; teloxantrone hydrochloride; temoporfin; teniposide; teroxirone; testolactone; thiamiprine; thioguanine; thiotepa; tiazofurin; tirapazamine; toremifene citrate; trestolone acetate; triciribine phosphate; trimetrexate; trimetrexate glucuronate; triptorelin; tubulozole hydrochloride; uracil mustard; uredepa; vapreotide; verteporfin; vinblastine sulfate; vincristine sulfate; vindesine; vindesine sulfate; vinepidine sulfate; vinglycinate sulfate; vinleurosine sulfate; vinorelbine tartrate; vinrosidine sulfate; vinzolidine sulfate; vorozole; zeniplatin; zinostatin; and zorubicin hydrochloride.

Other second agents include, but are not limited to: 20-epi-1,25 dihydroxyvitamin D3; 5-ethynyluracil; abiraterone; aclarubicin; acylfulvene; adecypenol; adozelesin; aldesleukin; ALL-TK antagonists; altretamine; ambamustine; amidox; amifostine; aminolevulinic acid; amrubicin; amsacrine; anagrelide; anastrozole; andrographolide; angiogenesis inhibitors; antagonist D; antagonist G; antarelix; anti-dorsalizing morphogenetic protein-1; antiandrogen, prostatic carcinoma; antiestrogen; antineoplaston; antisense oligonucleotides; aphidicolin glycinate; apoptosis gene modulators; apoptosis regulators; apurinic acid; ara-CDP-DL-PTBA; arginine deaminase; asulacrine; atamestane; atrimustine; axinastatin 1; axinastatin 2; axinastatin 3; azasetron; azatoxin; azatyrosine; baccatin III derivatives; balanol; batimastat; BCR/ABL antagonists; benzochlorins; benzoylstaurosporine; beta lactam derivatives; beta-alethine; betaclamycin B; betulinic acid; bFGF inhibitor; bicalutamide; bisantrene; bisaziridinylspermine; bisnafide; bistratene A; bizelesin; breflate; bropirimine; budotitane; buthionine sulfoximine; calcipotriol; calphostin C; camptothecin derivatives; capecitabine; carboxamide-amino-triazole; carboxyamidotriazole; CaRest M3; CARN700; cartilage derived inhibitor; carzelesin; casein kinase inhibitors (ICOS); castanospermine; cecropin B; cetrorelix; chlorins; chloroquinoxaline sulfonamide; cicaprost; cis-porphyrin; cladribine; clomifene analogues; clotrimazole; collismycin A; collismycin B; combretastatin A4; combretastatin analogue; conagenin; crambescidin 816; crisnatol; cryptophycin 8; cryptophycin A derivatives; curacin A; cyclopentanthraquinones; cycloplatam; cypemycin; cytarabine ocfosfate; cytolytic factor; cytostatin; dacliximab; decitabine; dehydrodidemnin B; deslorelin; dexamethasone; dexifosfamide; dexrazoxane; dexverapamil; diaziquone; didemnin B; didox; diethylnorspermine; dihydro-5-azacytidine; dihydrotaxol, 9-; dioxamycin; diphenyl spiromustine; docetaxel; docosanol; dolasetron; doxifluridine; doxorubicin; droloxifene; dronabinol; duocarmycin SA; ebselen; ecomustine; edelfosine; edrecolomab; eflornithine; elemene; emitefur; epirubicin; epristeride; estramustine analogue; estrogen agonists; estrogen antagonists; etanidazole; etoposide phosphate; exemestane; fadrozole; fazarabine; fenretinide; filgrastim; finasteride; flavopiridol; flezelastine; fluasterone; fludarabine; fluorodaunorubicin hydrochloride; forfenimex; formestane; fostriecin; fotemustine; gadolinium texaphyrin; gallium nitrate; galocitabine; ganirelix; gelatinase inhibitors; gemcitabine; glutathione inhibitors; hepsulfam; heregulin; hexamethylene bisacetamide; hypericin; ibandronic acid; idarubicin; idoxifene; idramantone; ilmofosine; ilomastat; imatinib (Gleevec^{®}), imiquimod; immunostimulant peptides; insulin-like growth factor-1 receptor inhibitor; interferon agonists; interferons; interleukins; iobenguane; iododoxorubicin; ipomeanol, 4-; iroplact; irsogladine; isobengazole; isohomohalicondrin B; itasetron; jasplakinolide; kahalalide F; lamellarin-N triacetate; lanreotide; leinamycin; lenograstim; lentinan sulfate; leptolstatin; letrozole; leukemia inhibiting factor; leukocyte alpha interferon; leuprolide+estrogen+progesterone; leuprorelin; levamisole; liarozole; linear polyamine analogue; lipophilic disaccharide peptide; lipophilic platinum compounds; lissoclinamide 7; lobaplatin; lombricine; lometrexol; lonidamine; losoxantrone; loxoribine; lurtotecan; lutetium texaphyrin; lysofylline; lytic peptides; maitansine; mannostatin A; marimastat; masoprocol; maspin; matrilysin inhibitors; matrix metal loproteinase inhibitors; menogaril; merbarone; meterelin; methioninase; metoclopramide; MIF inhibitor; mifepristone; miltefosine; mirimostim; mitoguazone; mitolactol; mitomycin analogues; mitonafide; mitotoxin fibroblast growth factor-saporin; mitoxantrone; mofarotene; molgramostim; Erbitux, human chorionic gonadotropin; monophosphoryl lipid A+myobacterium cell wall sk; mopidamol; mustard anticancer agent; mycaperoxide B; mycobacterial cell wall extract; myriaporone; N-acetyldinaline; N-substituted benzamides; nafarelin; nagrestip; naloxone+pentazocine; napavin; naphterpin; nartograstim; nedaplatin; nemorubicin; neridronic acid; nilutamide; nisamycin; nitric oxide modulators; nitroxide antioxidant; nitrullyn; oblimersen (Genasense^{®}); O6-benzylguanine; octreotide; okicenone; oligonucleotides; onapristone; ondansetron; oracin; oral cytokine inducer; ormaplatin; osaterone; oxaliplatin; oxaunomycin; paclitaxel; paclitaxel analogues; paclitaxel derivatives; palauamine; palmitoylrhizoxin; pamidronic acid; panaxytriol; panomifene; parabactin; pazelliptine; pegaspargase; peldesine; pentosan poly sulfate sodium; pentostatin; pentrozole; perflubron; perfosfamide; perillyl alcohol; phenazinomycin; phenylacetate; phosphatase inhibitors; picibanil; pilocarpine hydrochloride; pirarubicin; piritrexim; placetin A; placetin B; plasminogen activator inhibitor; platinum complex; platinum compounds; platinum-triamine complex; porfimer sodium; porfiromycin; prednisone; propyl bis-acridone; prostaglandin J2; proteasome inhibitors; protein A-based immune modulator; protein kinase C inhibitor; protein kinase C inhibitors, microalgal; protein tyrosine phosphatase inhibitors; purine nucleoside phosphorylase inhibitors; purpurins; pyrazoloacridine; pyridoxylated hemoglobin polyoxyethylene conjugate; raf antagonists; raltitrexed; ramosetron; ras farnesyl protein transferase inhibitors; ras inhibitors; ras-GAP inhibitor; retelliptine demethylated; rhenium-186 etidronate; rhizoxin; ribozymes; R2 retinamide; rohitukine; romurtide; roquinimex; rubiginone Bl; ruboxyl; safingol; saintopin; SarCNU; sarcophytol A; sargramostim; Sdi 1 mimetics; semustine; senescence derived inhibitor 1; sense oligonucleotides; signal transduction inhibitors; sizofiran; sobuzoxane; sodium borocaptate; sodium phenylacetate; solverol; somatomedin binding protein; sonermin; sparfosic acid; spicamycin D; spiromustine; splenopentin; spongistatin 1; squalamine; stipiamide; stromelysin inhibitors; sulfinosine; superactive vasoactive intestinal peptide antagonist; suradista; suramin; swainsonine; tallimustine; tamoxifen methiodide; tauromustine; tazarotene; tecogalan sodium; tegafur; tellurapyrylium; telomerase inhibitors; temoporfin; teniposide; tetrachlorodecaoxide; tetrazomine; thaliblastine; thiocoraline; thrombopoietin; thrombopoietin mimetic; thymalfasin; thymopoietin receptor agonist; thymotrinan; thyroid stimulating hormone; tin ethyl etiopurpurin; tirapazamine; titanocene bichloride; topsentin; toremifene; translation inhibitors; tretinoin; triacetyluridine; triciribine; trimetrexate; triptorelin; tropisetron; turosteride; tyrosine kinase inhibitors; tyrphostins; UBC inhibitors; ubenimex; urogenital sinus-derived growth inhibitory factor; urokinase receptor antagonists; vapreotide; variolin B; velaresol; veramine; verdins; verteporfin; vinorelbine; vinxaltine; vitaxin; vorozole; zanoterone; zeniplatin; zilascorb; and zinostatin stimalamer.

Specific second active agents include, but are not limited to, 2-methoxyestradiol, telomestatin, inducers of apoptosis in mutiple myeloma cells (such as, for example, TRAIL), statins, semaxanib, cyclosporin, etanercept, doxycycline, bortezomib, oblimersen (Genasense 1 remicade, docetaxel, celecoxib, melphalan, dexamethasone (Decadron^{®}), steroids, gemcitabine, cisplatinum, temozolomide, etoposide, cyclophosphamide, temodar, carboplatin, procarbazine, gliadel, tamoxifen, topotecan, methotrexate, Arisa^{®}, taxol, taxotere, fluorouracil, leucovorin, irinotecan, xeloda, CPT-1 1, interferon alpha, pegylated interferon alpha (e.g., PEG INTRON-A), capecitabine, cisplatin, thiotepa, fludarabine, carboplatin, liposomal daunorubicin, cytarabine, doxetaxol, paclitaxel, vinblastine, IL-2, GM-CSF, dacarbazine, vinorelbine, zoledronic acid, palmitronate, biaxin, busulphan, prednisone, bisphosphonate, arsenic trioxide, vincristine, doxorubicin (Doxil^{®}), paclitaxel, ganciclovir, adriamycin, estramustine sodium phosphate (Emcyt^{®}), sulindac, and etoposide.

Specific second active agents include, but are not limited to, regorafenib, ziv-aflibercept, and tipiracil/trifluridine for the treatment of colorectal cancer; romidepsin for the treatment of peripheral T-cell lymphoma and cutaneous T-cell lymphoma; brentuximab vedotin for the treatment of Hodgkin's lymphoma and anaplastic large cell lymphoma; pixantrone and rituximab for the treatment of non-Hodgkin's lymphoma; idelalisib for the treatment of chronic lymphocytic leukemia, follicular lymphoma, and small lymphocytic lymphoma; mogamulizumab for the treatment of angioimmunoblastic T-cell lymphoma; belinostat for the treatment of peripheral T-cell lymphoma; ibrutinib for the treatment of mantle cell lymphoma and Waldenstrom's macroglobulinemia; bortezomib for the treatment of mantle cell lymphoma; chidamide for the treatment of peripheral T-cell lymphoma; nivolumab for the treatment of Hodgkin's lymphoma; siltuximab for the treatment of Castleman's disease; olaparib, bevacizumab, and rucaparib for the treatment of ovarian cancer; bevacizumab for the treatment of cervical cancer; vismodegib and sonidegib for the treatment of basal cell carcinoma; ruxolitinib for the treatment of myeloproliferative disorders; ramucirumab for the treatment of gastric cancer; carfilzomib, pomalidomide, daratumumab, ixazomib, panobinostat, and elotuzumab for the treatment of multiple myeloma; abiraterone acetate, enzalutamide, and radium-223 dichloride for the treatment of prostate cancer; mifamurtide, trabectedin, eribulin, and olaratumab for the treatment of sarcoma; crizotinib, afatinib, alectinib, ceritinib, ramucirumab, nivolumab, pembrolizumab, necitumumab, osimertinib, gefitinib, and atezolizumab for the treatment of lung cancer; bosutinib, omacetaxine mepesuccinate, and radotinib for the treatment of chronic myelogenous leukemia; obinutuzumab for the treatment of chronic lymphocytic leukemia and follicular lymphoma; ponatinib for the treatment of chronic myelogenous leukemia and acute lymphoblastic leukemia; blinatumomab for the treatment of acute lymphoblastic leukemia; ibrutinib, ofatumumab, and venetoclax for the treatment of chronic lymphocytic leukemia; regorafenib for the treatment of gastrointestinal stromal tumors; vandetanib, cabozantinib and lenvatinib for the treatment of thyroid cancer; dinutuximab for the treatment of neuroblastoma; atezolizumab for the treatment of bladder cancer; nivolumab and pembrolizumab for the treatment of head and neck cancer; irinotecan liposome for the treatment of pancreatic cancer; axitinib, nivolumab, lenvatinib and cabozantinib for the treatment of kidney cancer; pertuzumab, ado-trastuzumab emtansine, and palbociclib for the treatment of breast cancer; and ipilimumab, vemurafenib, trametinib, dabrafenib, pembrolizumab, nivolumab, cobimetinib, and T-Vec for the treatment of melanoma.

The amount of a CCR5 inhibitor provided herein administered according to the present invention will vary, as recognized by those skilled in the art, dependent on route of administration, excipient usage, and the possibility of co-usage with other therapeutic treatments including use of other therapeutic agents.

According to some embodiments, a CCR5 inhibitor described herein may be administered in an amount of from about 0.1 mg to about 400 mg. According to some embodiments, a CCR5 inhibitor provided herein may be administered in an amount of from about 1mg to about 100 mg. According to some embodiments, a CCR5 inhibitor provided herein may be administered in an amount of from about 4 mg to about 40 mg.

According to some embodiments, a CCR5 inhibitor provided herein may be administered in an amount of from about 0.1 mg to about 300 mg. According to some embodiments, a CCR5 inhibitor provided herein may be administered in an amount of from about 0.1 mg to about 200 mg. According to some embodiments, a CCR5 inhibitor provided herein may be administered in an amount of from about 0.1 mg to about 100 mg. According to some embodiments, a CCR5 inhibitor provided herein may be administered in an amount of from about 0.1 mg to about 95 mg. According to some embodiments, a CCR5 inhibitor provided herein may be administered in an amount of from about 0.1 mg to about 90 mg. According to some embodiments, a CCR5 inhibitor provided herein may be administered in an amount of from about 0.1 mg to about 85 mg. According to some embodiments, a CCR5 inhibitor provided herein may be administered in an amount of from about 0.1 mg to about 80 mg. According to some embodiments, a CCR5 inhibitor provided herein may be administered in an amount of from about 0.1 mg to about 75 mg. According to some embodiments, a CCR5 inhibitor provided herein may be administered in an amount of from about 0.1 mg to about 70 mg. According to some embodiments, a CCR5 inhibitor provided herein may be administered in an amount of from about 0.1 mg to about 65 mg. According to some embodiments, a CCR5 inhibitor provided herein may be administered in an amount of from about 0.1 mg to about 60 mg. According to some embodiments, a CCR5 inhibitor provided herein may be administered in an amount of from about 0.1 mg to about 55 mg. According to some embodiments, a CCR5 inhibitor provided herein may be administered in an amount of from about 0.1 mg to about 50 mg. According to some embodiments, a CCR5 inhibitor provided herein may be administered in an amount of from about 0.1 mg to about 45 mg. According to some embodiments, a CCR5 inhibitor provided herein may be administered in an amount of from about 0.1 mg to about 40 mg. According to some embodiments, a CCR5 inhibitor provided herein may be administered in an amount of from about 0.1 mg to about 35 mg. According to some embodiments, a CCR5 inhibitor provided herein may be administered in an amount of from about 0.1 mg to about 30 mg. According to some embodiments, a CCR5 inhibitor provided herein may be administered in an amount of from about 0.1 mg to about 25 mg. According to some embodiments, a CCR5 inhibitor provided herein may be administered in an amount of from about 0.1 mg to about 20 mg. According to some embodiments, a CCR5 inhibitor provided herein may be administered in an amount of from about 0.1 mg to about 15 mg. According to some embodiments, a CCR5 inhibitor provided herein may be administered in an amount of from about 0.1 mg to about 10 mg. According to some embodiments, a CCR5 inhibitor provided herein may be administered in an amount of from about 0.1 mg to about 5 mg. According to some embodiments, a CCR5 inhibitor provided herein may be administered in an amount of from about 0.1 mg to about 1 mg.

According to some embodiments, a CCR5 inhibitor provided herein may be administered in an amount of from about 1 mg to about 400 mg. According to some embodiments, a CCR5 inhibitor provided herein may be administered in an amount of from about 1 mg to about 300 mg. According to some embodiments, a CCR5 inhibitor provided herein may be administered in an amount of from about 1 mg to about 200 mg. According to some embodiments, a CCR5 inhibitor provided herein may be administered in an amount of from about 1 mg to about 95 mg. According to some embodiments, a CCR5 inhibitor provided herein may be administered in an amount of from about 1 mg to about 90 mg. According to some embodiments, a CCR5 inhibitor provided herein may be administered in an amount of from about 1 mg to about 85 mg. According to some embodiments, a CCR5 inhibitor provided herein may be administered in an amount of from about 1 mg to about 80 mg. According to some embodiments, a CCR5 inhibitor provided herein may be administered in an amount of from about 1 mg to about 75 mg. According to some embodiments, a CCR5 inhibitor provided herein may be administered in an amount of from about 1 mg to about 70 mg. According to some embodiments, a CCR5 inhibitor provided herein may be administered in an amount of from about 1 mg to about 65 mg. According to some embodiments, a CCR5 inhibitor provided herein may be administered in an amount of from about 1 mg to about 60 mg. According to some embodiments, a CCR5 inhibitor provided herein may be administered in an amount of from about 1 mg to about 55 mg. According to some embodiments, a CCR5 inhibitor provided herein may be administered in an amount of from about 1 mg to about 50 mg. According to some embodiments, a CCR5 inhibitor provided herein may be administered in an amount of from about 1 mg to about 45 mg. According to some embodiments, a CCR5 inhibitor provided herein may be administered in an amount of from about 1 mg to about 40 mg. According to some embodiments, a CCR5 inhibitor provided herein may be administered in an amount of from about 1 mg to about 35 mg. According to some embodiments, a CCR5 inhibitor provided herein may be administered in an amount of from about 1 mg to about 30 mg. According to some embodiments, a CCR5 inhibitor provided herein may be administered in an amount of from about 1 mg to about 25 mg. According to some embodiments, a CCR5 inhibitor provided herein may be administered in an amount of from about 1 mg to about 20 mg. According to some embodiments, a CCR5 inhibitor provided herein may be administered in an amount of from about 1 mg to about 15 mg. According to some embodiments, a CCR5 inhibitor provided herein may be administered in an amount of from about 1 mg to about 10 mg. According to some embodiments, a CCR5 inhibitor described herein may be administered in an amount of from about 1 mg to about 5 mg.

According to some embodiments, a CCR5 inhibitor provided herein may be administered in an amount of from about 5 mg to about 400 mg. According to some embodiments, a CCR5 inhibitor provided herein may be administered in an amount of from about 5 mg to about 300 mg. According to some embodiments, a CCR5 inhibitor provided herein may be administered in an amount of from about 5 mg to about 200 mg. According to some embodiments, a CCR5 inhibitor provided herein may be administered in an amount of from about 5 mg to about 100 mg. According to some embodiments, a CCR5 inhibitor provided herein may be administered in an amount of from about 5 mg to about 95 mg. According to some embodiments, a CCR5 inhibitor provided herein may be administered in an amount of from about 5 mg to about 90 mg. According to some embodiments, a CCR5 inhibitor provided herein may be administered in an amount of from about 5 mg to about 85 mg. According to some embodiments, a CCR5 inhibitor provided herein may be administered in an amount of from about 5 mg to about 80 mg. According to some embodiments, a CCR5 inhibitor provided herein may be administered in an amount of from about 5 mg to about 75 mg. According to some embodiments, a CCR5 inhibitor provided herein may be administered in an amount of from about 5 mg to about 70 mg. According to some embodiments, a CCR5 inhibitor provided herein may be administered in an amount of from about 5 mg to about 65 mg. According to some embodiments, a CCR5 inhibitor provided herein may be administered in an amount of from about 5 mg to about 60 mg. According to some embodiments, a CCR5 inhibitor provided herein may be administered in an amount of from about 5 mg to about 55 mg. According to some embodiments, a CCR5 inhibitor provided herein may be administered in an amount of from about 5 mg to about 50 mg. According to some embodiments, a CCR5 inhibitor provided herein may be administered in an amount of from about 5 mg to about 45 mg. According to some embodiments, a CCR5 inhibitor provided herein may be administered in an amount of from about 5 mg to about 40 mg. According to some embodiments, a CCR5 inhibitor provided herein may be administered in an amount of from about 5 mg to about 35 mg. According to some embodiments, a CCR5 inhibitor provided herein may be administered in an amount of from about 5 mg to about 30 mg. According to some embodiments, a CCR5 inhibitor provided herein may be administered in an amount of from about 5 mg to about 25 mg. According to some embodiments, a CCR5 inhibitor provided herein may be administered in an amount of from about 5 mg to about 20 mg. According to some embodiments, a CCR5 inhibitor provided herein may be administered in an amount of from about 5 mg to about 15 mg. According to some embodiments, a CCR5 inhibitor provided herein may be administered in an amount of from about 5 mg to about 10 mg.

According to some embodiments, a CCR5 inhibitor provided herein may be administered in an amount of from about 10 mg to about 400 mg. According to some embodiments, a CCR5 inhibitor provided herein may be administered in an amount of from about 10 mg to about 300 mg. According to some embodiments, a CCR5 inhibitor provided herein may be administered in an amount of from about 10 mg to about 200 mg. According to some embodiments, a CCR5 inhibitor provided herein may be administered in an amount of from about 10 mg to about 100 mg. According to some embodiments, a CCR5 inhibitor provided herein may be administered in an amount of from about 10 mg to about 95 mg. According to some embodiments, a CCR5 inhibitor provided herein may be administered in an amount of from about 10 mg to about 90 mg. According to some embodiments, a CCR5 inhibitor provided herein may be administered in an amount of from about 10 mg to about 85 mg. According to some embodiments, a CCR5 inhibitor provided herein may be administered in an amount of from about 10 mg to about 80 mg. According to some embodiments, a CCR5 inhibitor provided herein may be administered in an amount of from about 10 mg to about 75 mg. According to some embodiments, a CCR5 inhibitor provided herein may be administered in an amount of from about 10 mg to about 70 mg. According to some embodiments, a CCR5 inhibitor provided herein may be administered in an amount of from about 10 to about 65 mg. According to some embodiments, a CCR5 inhibitor provided herein may be administered in an amount of from about 10 mg to about 60 mg. According to some embodiments, a CCR5 inhibitor provided herein may be administered in an amount of from about 10 mg to about 55 mg. According to some embodiments, a CCR5 inhibitor provided herein may be administered in an amount of from about 10 mg to about 50 mg. According to some embodiments, a CCR5 inhibitor provided herein may be administered in an amount of from about 10 mg to about 45 mg. According to some embodiments, a CCR5 inhibitor provided herein may be administered in an amount of from about 10 mg to about 40 mg. According to some embodiments, a CCR5 inhibitor provided herein may be administered in an amount of from about 10 mg to about 35 mg. According to some embodiments, a CCR5 inhibitor provided herein may be administered in an amount of from about 10 mg to about 30 mg. According to some embodiments, a CCR5 inhibitor provided herein may be administered in an amount of from about 10 mg to about 25 mg. According to some embodiments, a CCR5 inhibitor provided herein may be administered in an amount of from about 10 mg to about 20 mg. According to some embodiments, a CCR5 inhibitor provided herein may be administered in an amount of from about 10 mg to about 15 mg.

According to some embodiments, a CCR5 inhibitor provided herein may be administered in an amount of about 400 mg, about 350 mg, about 300 mg, about 250 mg, about 200 mg, about 150 mg, about 100 mg, about 95 mg, about 90 mg, about 85 mg, about 80 mg, about 75 mg, about 70 mg, about 65 mg, about 60 mg, about 55 mg, about 50 mg, about 45 mg, about 40 mg, about 35 mg, about 30 mg, about 25 mg, about 20 mg, about 19 mg, about 18 mg, about 17 mg, about 16 mg, about 15 mg, about 14 mg, about 13 mg, about 12 mg, about 11 mg, about 10 mg, about 9 mg, about 8 mg, about 7 mg, about 6 mg, about 5 mg, about 4 mg, about 3 mg, about 2 mg, about 1 mg, about 0.5 mg, or about 0.1 mg.

According to some embodiments, a CCR5 inhibitor, pharmaceutical composition or dosage form provided herein may be administered once a day or in separate administrations of 2, 3, 4, 5 or 6 equal doses per day. According to other embodiments, a CCR5 inhibitor, pharmaceutical composition or dosage form provided herein may be administered twice, thrice, four times, five times, six times per day, or more.

According to some embodiments, a CCR5 inhibitor, pharmaceutical composition or dosage form provided herein may be administered chronically. According to some embodiments, a CCR5 inhibitor, pharmaceutical composition, or dosage form provided herein may be administered for one, two, three, four, five, six, or seven days; for one, two, three, or four weeks; for one, two, three, four, five, six, seven, eight, nine, ten, eleven, twelve months or longer.

For chronical administration, a CCR5 inhibitor, pharmaceutical composition or dosage form provided herein may be administered at a frequency of daily, six times a week, five times a week, four times a week, three times a week, twice a week, once a week, once every two weeks, once every three weeks, once every four weeks, once every five weeks, once every six weeks, once every seven weeks, once every eight weeks, once every nine weeks, once every ten weeks, twice a month, once a month, once every two months, or once every three months.

In some embodiments, a CCR5 inhibitor, pharmaceutical composition or dosage form provided herein may be administered or provided for administration separately, sequentially or simultaneously in combination with a further pharmacologically active compound. In some embodiments, a CCR5 inhibitor, pharmaceutical composition or dosage form provided herein is used in conjunction with a further pharmacologically active compound. Alternatively, the therapeutic administration of a CCR5 inhibitor, pharmaceutical composition or dosage form provided herein may precede or follow the further pharmacologically active compound treatment by intervals ranging from minutes to weeks. In embodiments where the CCR5 inhibitor, pharmaceutical composition or dosage form provided herein and the further pharmacologically active compound are administered separately, one would generally ensure that a significant period of time did not expire between each delivery, such that the CCR5 inhibitor, pharmaceutical composition or dosage form provided herein and the pharmacologically active compound would still be able to exert an advantageously combined effect on the subject. In such instances, it is contemplated that one may administer both modalities within about 12-24 h of each other and, more preferably, within about 6-12 h of each other. In some situations, it may be desirable to extend the time period for administration significantly, where several days (2, 3, 4, 5, 6 or 7) to several weeks (1, 2, 3, 4, 5, 6, 7 or 8) lapse between the respective administrations.

A CCR5 inhibitor, pharmaceutical composition, or dosage form described herein may be administered until the subject in need thereof does not require treatment, prophylaxis, or amelioration of cancer.

It should be understood, however, that a specific dosage and treatment regimen for any particular subject will depend upon a variety of factors, including the activity of the specific compound employed, the age, body weight, general health, sex, diet, time of administration, rate of excretion, drug combination, and the judgment of the treating physician and the severity of the particular disorder being treated. The amount of a CCR5 inhibitor can also depend upon the therapeutic or prophylactic agent, if any, with which the CCR5 inhibitor is co-administered.

### EXAMPLES

### Example 1: 5P12-RANTES is more potent than several other CCR5 inhibitors at inhibiting signaling through human CCR5.

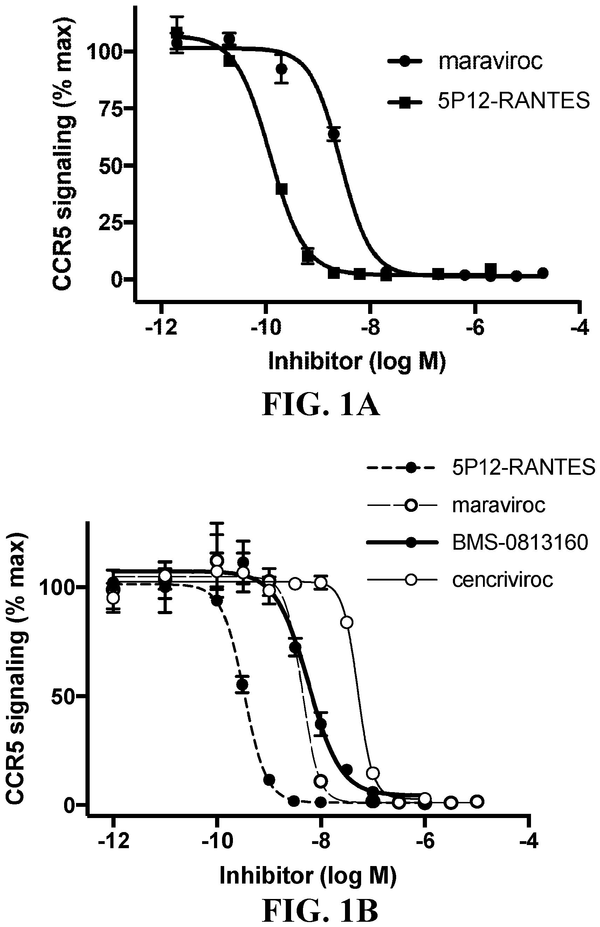

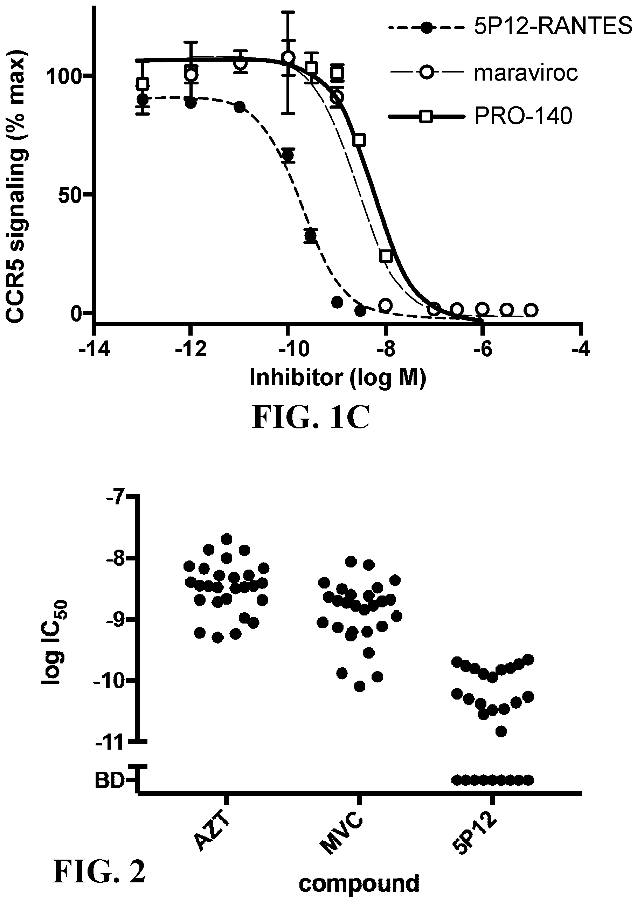

Chinese hamster ovary cells engineered to express human CCR5 plus an aequorin calcium sensor to detect receptor signaling activity were treated with the indicated inhibitors at increasing concentrations, followed by a natural CCR5 agonist, human MIP-1β/CCL4. The signaling activity measured in the cells at each inhibitor concentration is expressed as a percentage of the signal elicited by the agonist in the absence of inhibitor (n=2) (Figures 1A, 1B and 1C). In Figure 1A, fitted dose-inhibition curves (GraphPadPrism) indicated IC₅₀ values of 2.7 nM and 0.12 nM for maraviroc and 5P12-RANTES, respectively. In Figure 1B, fitted dose-inhibition curves (GraphPadPrism) indicated IC₅₀ values of 51 nM, 5.7 nM, 4.3 nM and 0.3 nM for cencriviroc, BMS-0813160, maraviroc and 5P12-RANTES, respectively. In Figure 1C, fitted dose-inhibition curves (GraphPadPrism) indicated IC₅₀ values of 5.6 nM, 2.6 nM and 0.2 nM for PRO-140, maraviroc and 5P12-RANTES, respectively.

### Example 2: Comparison of antiretroviral potency of 5P12-RANTES with MVC

The antiretroviral potency (log IC₅₀) of 5P12-RANTES against HIV-1_{BaL} was compared with that of the reference compounds AZT and maraviroc (MVC) in a replication assay using peripheral bloodmononuclear cells from a panel of US donors representing Caucasian, Black, Asian and Latino ethnicities (Figure 2). For each donor, each compound was evaluated at 9 concentrations in triplicate. Each point represents log IC₅₀ for an individual donor. BD: below (i.e. more potent than) assay detection limit. These data suggest that 5P12-RANTES is 10-30 times more potent than MVC in inhibiting HIV-1 replication.

### Example 3: Determination of inhibitory activity of 5P12-RANTES on human CCR1 and CCR3

5P12-RANTES was tested for inhibitory activity on recombinant human CCR1 (FAST-058G) and CCR3 (FAST-061G) receptors using GTPγ³⁵S functional assays in dose response and in duplicate.

Assay buffer was 20mM HEPES pH 7.4; 200mM NaCl, 10µg/ml saponin, MgCl₂ at optimized concentration for the specific receptor, 0.1% BSA. CHO-K1 cells expressing recombinant human receptor membrane extracts were thawed on ice, diluted in assay buffer and kept on ice. GDP was diluted in assay buffer to give optimized concentration for the specific receptor. Beads were PVT-WGA (PerkinElmer, RPNQ001), diluted in assay buffer at a concentration optimized for the specific receptor. GTPγ³⁵S (PerkinElmer NEG030X) was diluted in assay buffer to give 0.1 nM.

For inhibition testing, membranes were mixed with GDP and incubated for at least 15 min on ice. In parallel, GTPγ³⁵S was mixed with the beads just before starting the reaction. The following reagents were successively added in the wells of an Optiplate (PerkinElmer): 50 µL of test or reference ligand, 20 µL of the membranes: GDP mix, 10 µL of reference agonist at historical EC₈₀ and 20 µL of the GTPγ³⁵S:beads mix.

The plates were covered with a top seal, mixed on an orbital shaker for 2 min, and then incubated for 1 hour at room temperature. Then the plates were centrifuged for 10 min at 2000 rpm, incubated at room temperature for 1 hour and counted for 1 min/well with a PerkinElmer TopCount reader.

On each day of experimentation and prior to the testing of compounds, reference compounds (J113863 for CCR1; UCB35625 for CCR3) were tested at several concentrations in duplicate (n=2) to obtain a dose-response curve and an estimated EC₅₀/IC₅₀ value.

Reference values thus obtained for the test were compared to historical values obtained from the same receptor (4.89 nM for CCR1; 213 nM for CCR3) and used to validate the experimental session. A session was considered valid only if the reference value was found to be within a 0.5-log interval from the historical value.

For replicate determinations, the maximum variability tolerated in the test was of +/-20% around the average of the replicates.

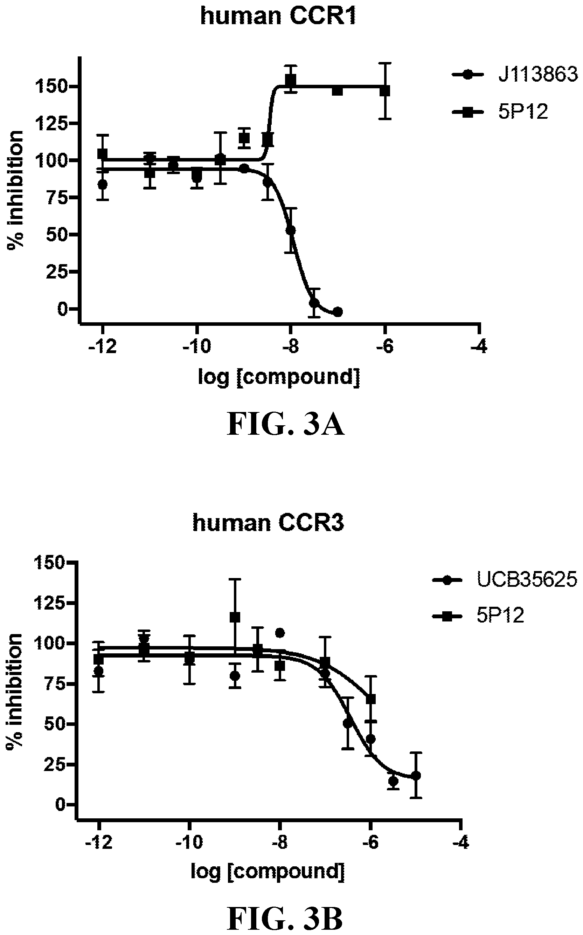

Dose-response data were analyzed with XLfit (IDBS) software using nonlinear regression applied to a sigmoidal dose-response model. At the highest concentrations, SP12-RANTES showed a moderate level of potentiation of CCR1 signaling in this assay, but no antagonism (Figure 3A). 5P12-RANTES also showed no antagonism towards CCR3 (Figure 3 B).

### Example 4: Efficacy of SP12-RANTES in a colorectal cancer animal model

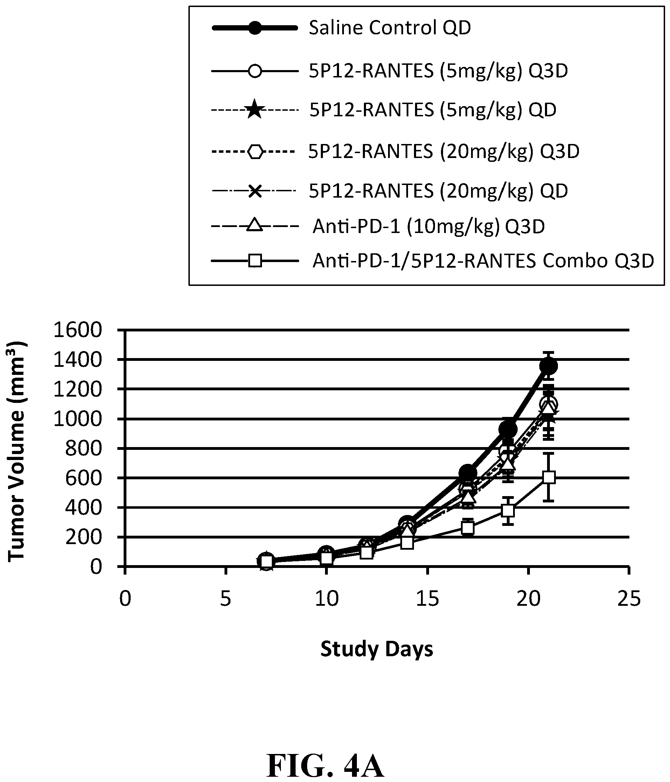

In one experiment, BALB/c mice were inoculated subcutaneously with the CT-26 colorectal cancer cell line. Three days after inoculation intraperitoneal treatment was started with 5P12-RANTES, a murine anti-PD-1 antibody, 5P12-RANTES + anti-PD-1, or saline placebo. Treatment was given daily or every third day. As shown in Figure 4A, treatment with SP12-RANTES alone at led to statistically significant delayed tumor growth at multiple time-points, and an even more profound effect on the decrease in mean tumor volume was observed in combination cohort where animals received both 5P12-RANTES and the anti-PD-1 antibody (p>0.05). No significant adverse events were observed during the course of treatment.

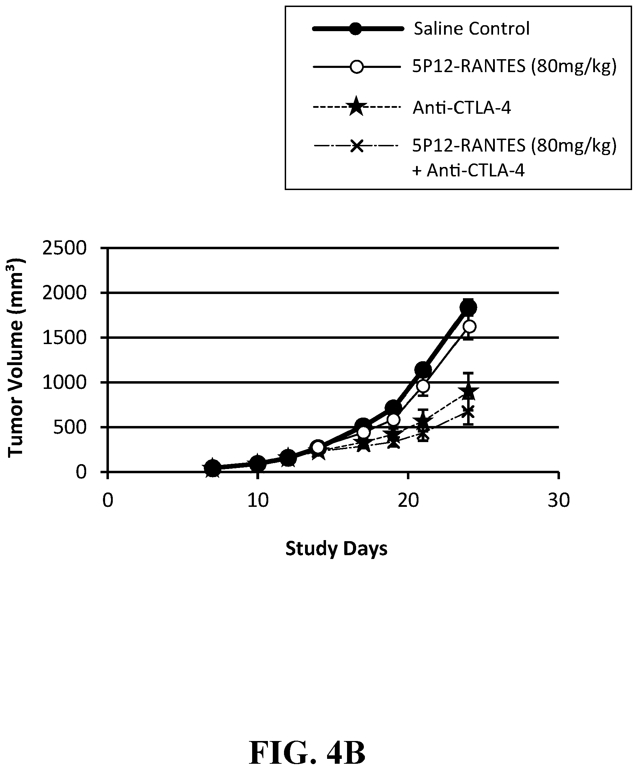

In another experiment, BALB/c mice were inoculated subcutaneously with the CT-26 colorectal cancer cell line. Ten days after inoculation intraperitoneal treatment was started with 5P12-RANTES, an anti-CTLA-4 antibody, 5P12-RANTES + anti-CTLA-4, or saline placebo. 5P12-RANTES treatment was given five times a week. As shown in Figure 4B, treatment with 5P12-RANTES alone at led to delayed tumor growth at multiple time-points, and an even more profound effect on the decrease in mean tumor volume was observed in combination cohort where animals received both SP12-RANTES and the anti-CTLA-4 antibody. No adverse events were observed during the course of treatment.

The citation of any publication is for its disclosure prior to the filing date and should not be construed as an admission that the present invention is not entitled to antedate such publication by virtue of prior invention.

It is to be understood that any numerical value inherently contains certain errors necessarily resulting from the standard deviation found in the respective testing measurements. Also, as used herein, the term "about" generally means within 10%, 5%, 1%, or 0.5% of a given value or range. Alternatively, the term "about" means within an acceptable standard error of the mean when considered by one of ordinary skill in the art. Unless indicated to the contrary, the numerical parameters set forth in the present disclosure and attached claims are approximations that can vary as desired. At the very least, each numerical parameter should at least be construed in light of the number of reported significant digits and by applying ordinary rounding techniques.

It must be noted that as used in this specification and the appended claims, the singular forms "a," "an," and "the" include plural reference unless the context clearly dictates otherwise. Unless defined otherwise all technical and scientific terms used herein have the same meaning as commonly understood to one of ordinary skill in the art to which this invention belongs.

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified. Thus, as a non-limiting example, a reference to "A and/or B", when used in conjunction with open-ended language such as "comprising" can refer, in one embodiment, to A only (optionally including elements other than B); in another embodiment, to B only (optionally including elements other than A); in yet another embodiment, to both A and B (optionally including other elements); etc.

As used herein in the specification and in the claims, "or" should be understood to encompass the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items.

As used herein, whether in the specification or the appended claims, the transitional terms "comprising", "including", "carrying", "having", "containing", "involving", and the like are to be understood as being inclusive or open-ended (i.e., to mean including but not limited to), and they do not exclude unrecited elements, materials or method steps. Only the transitional phrases "consisting of" and "consisting essentially of", respectively, are closed or semi-closed transitional phrases with respect to claims and exemplary embodiment paragraphs herein. The transitional phrase "consisting of" excludes any element, step, or ingredient which is not specifically recited. The transitional phrase "consisting essentially of" limits the scope to the specified elements, materials or steps and to those that do not materially affect the basic characteristic(s) of the invention disclosed and/or claimed herein.

## Claims

1. A CCR5 inhibitor for use in treating cancer in a subject, wherein the CCR5 inhibitor comprises an N-terminal portion and a C-terminal portion, wherein the N-terminal portion comprises the signature sequence QGP[P or L] and the amino acid sequence of the C-terminal portion is at least 70% identical to SEQ ID NO: 1, and wherein the CCR5 inhibitor inhibits HIV entry into cells and is selective for CCR5 over CCR1 and CCR3.

2. The CCR5 inhibitor for use according to claim 1, wherein the CCR5 inhibitor inhibits only a subset of CCR5 intracellular signaling pathways.

3. The CCR5 inhibitor for use according to claim 2, wherein the CCR5 inhibitor inhibits or reduces the inflammatory effects of CCR5, for example, leads to a signaling response of 30% or less of the maximum response (Emax) elicited by PSC-RANTES, when tested at a concentration of 300 nM in a Calcium Flux signaling assay.

4. The CCR5 inhibitor for use according to claim 1, wherein the signature sequence is QGP[P or L] [L or G or S or M] [M or D or S or Q or G].

5. The CCR5 inhibitor for use according to claim 1, wherein the signature sequence is QGP[P or L][L or G or S or M][M or D or S or Q or G]XX[Q or G or L or A or T or S]X, wherein X denotes any natural or modified amino acid.

6. The CCR5 inhibitor for use according to claim 1, wherein the signature sequence is QGP[P or L]LM or QGPPG[D or S].

7. The CCR5 inhibitor for use according to claim 1, wherein the signature sequence is QGPPLM or QGPPGD.

8. The CCR5 inhibitor for use according to claim 1, wherein the signature sequence is QGP[P or L][L or M][M or Q][A or W or G or Q or N]X[Q or G or L][S or V or T or G], wherein X denotes any natural or modified amino acid.

9. The CCR5 inhibitor for use according to claim 1, wherein the signature sequence is QGPPLM[A or W][L or T or M][Q or G][S or V or T or G].

10. The CCR5 inhibitor for use according to claim 1, wherein the signature sequence is QGPP[G or L][M or Q]XX[Q or S][S or V], wherein X denotes any natural or modified amino acid.

11. The CCR5 inhibitor for use according to claim 1, wherein the signature sequence is selected from the group QGPPLMALQS (SEQ ID NO: 2), QGPPLMWMQV (SEQ ID NO: 3), QGPPLMWLQV (SEQ ID NO: 4), QGPPLMWTQS (SEQ ID NO: 5), QGPPLMWLQT (SEQ ID NO: 6), QGPPLMWTQV (SEQ ID NO: 7), QGPPLMWMQS (SEQ ID NO: 8), QGPPLMATQS (SEQ ID NO: 9), QGPPLMWLQS (SEQ ID NO: 10), QGPPLMALQV (SEQ ID NO: 11), QGPPLMWLGG (SEQ ID NO: 12), QGPPLMWRGS (SEQ ID NO: 13), QGPLLMWLQV (SEQ ID NO: 14), QGPPLMQTTP (SEQ ID NO: 15), QGPPLSWLQV (SEQ ID NO: 30), QGPPLSWLQS (SEQ ID NO: 31), QGPPGQWSQV (SEQ ID NO: 32), QGPPMMAGLS (SEQ ID NO: 33), QGPPLSWQQS (SEQ ID NO: 34), QGPPGMWSQS (SEQ ID NO: 35), QGPPLQWRQS (SEQ ID NO: 36), QGPPLMGTQS (SEQ ID NO: 37), QGPPLMQLQV (SEQ ID NO: 38), QGPPLSWSQV (SEQ ID NO: 39), QGPPMSWSQS (SEQ ID NO: 40), QGPPLMNLQV (SEQ ID NO: 41), QGPPMSAYQV (SEQ ID NO: 42) and QGPPMQGGLS (SEQ ID NO: 43).

12. The CCR5 inhibitor for use according to claim 1, wherein the signature sequence is selected from the group QGPPLMALQS (SEQ ID NO: 2), QGPPLMWMQV (SEQ ID NO: 3), QGPPLMWLQV (SEQ ID NO: 4), QGPPLMWTQS (SEQ ID NO: 5), QGPPLMWLQT (SEQ ID NO: 6), QGPPLMWTQV (SEQ ID NO: 7), QGPPLMWMQS (SEQ ID NO: 8), QGPPLMATQS (SEQ ID NO: 9), QGPPLMWLQS (SEQ ID NO: 10), QGPPLMALQV (SEQ ID NO: 11), QGPPLMWLGG (SEQ ID NO: 12), QGPPLMWRGS (SEQ ID NO: 13), QGPLLMWLQV (SEQ ID NO: 14) and QGPPLMQTTP (SEQ ID NO: 15).

13. The CCR5 inhibitor for use according to claim 1, wherein the signature sequence is QGPPLMATQS (SEQ ID NO: 9).

14. The CCR5 inhibitor for use according to claim 13, wherein the signature sequence is located at the extreme N-terminus.

15. The CCR5 inhibitor for use according to claim 14, wherein the C-terminal portion is identical to SEQ ID NO: 1.

16. The CCR5 inhibitor for use according to claim 1, wherein the CCR5 inhibitor comprises the amino acid sequence of SEQ ID NO: 70.

17. The CCR5 inhibitor for use according to any one of claims 1 to 16, wherein the cancer is colorectal cancer, breast cancer, lung cancer, prostate cancer, ovarian cancer, pancreatic cancer, esophageal cancer, gastric cancer, liver cancer, or leukemia.

18. The CCR5 inhibitor for use according to claim 17, wherein the cancer is metastatic.

19. The CCR5 inhibitor for use according to claim 17, wherein the cancer is refractory and/or resistant to chemotherapy or radiation.

## Patentansprüche

1. Ein CCRS-Inhibitor zur Verwendung bei der Behandlung von Krebs in einem Subjekt, wobei der CCRS-Inhibitor einen N-terminalen Abschnitt und einen C-terminalen Abschnitt umfasst, wobei der N-terminale Abschnitt die Signatursequenz QGP[P oder L] umfasst und die Aminosäuresequenz des C-terminalen Abschnitts zu mindestens 70 % mit SEQ ID NO: 1 identisch ist, und wobei der CCRS-Inhibitor den HIV-Eintritt in Zellen hemmt und für CCR5 gegenüber CCR1 und CCR3 selektiv ist.

2. Der CCRS-Inhibitor zur Verwendung nach Anspruch 1, wobei der CCRS-Inhibitor nur eine Teilmenge der intrazellulären CCR5-Signalwege hemmt.

3. Der CCRS-Inhibitor zur Verwendung nach Anspruch 2, wobei der CCRS-Inhibitor die entzündlichen Wirkungen von CCR5 hemmt oder reduziert, beispielsweise zu einer Signalantwort von 30 % oder weniger der durch PSC-RANTES ausgelösten maximalen Antwort (Emax) führt, wenn er bei einer Konzentration von 300 nM in einem Calciumfluss-Signaltest getestet wird.

4. Der CCRS-Inhibitor zur Verwendung nach Anspruch 1, wobei die Signatursequenz QGP[P oder L] [L oder G oder S oder M] [M oder D oder S oder Q oder G] ist.

5. Der CCRS-Inhibitor zur Verwendung nach Anspruch 1, wobei die Signatursequenz QGP[P oder L][L oder G oder S oder M][M oder D oder S oder Q oder G]XX[Q oder G oder L oder A oder T oder S]X ist, wobei X eine beliebige natürliche oder modifizierte Aminosäure bezeichnet.

6. Der CCRS-Inhibitor zur Verwendung nach Anspruch 1, wobei die Signatursequenz QGP[P oder L]LM oder QGPPG[D oder S] ist.

7. Der CCRS-Inhibitor zur Verwendung nach Anspruch 1, wobei die Signatursequenz QGPPLM oder QGPPGD ist.

8. Der CCRS-Inhibitor zur Verwendung nach Anspruch 1, wobei die Signatursequenz QGP[P oder L][L oder M][M oder Q][A oder W oder G oder Q oder N]X[Q oder G oder L][S oder V oder T oder G] ist, wobei X eine beliebige natürliche oder modifizierte Aminosäure bezeichnet.

9. Der CCRS-Inhibitor zur Verwendung nach Anspruch 1, wobei die Signatursequenz QGPPLM[A oder W][L oder T oder M][Q oder G][S oder V oder T oder G] ist.

10. Der CCRS-Inhibitor zur Verwendung nach Anspruch 1, wobei die Signatursequenz QGPP[G oder L][M oder Q]XX[Q oder S][S oder V] ist, wobei X eine beliebige natürliche oder modifizierte Aminosäure bezeichnet.

11. Der CCRS-Inhibitor zur Verwendung nach Anspruch 1, wobei die Signatursequenz ausgewählt ist aus der Gruppe QGPPLMALQS (SEQ ID NO: 2), QGPPLMWMQV (SEQ ID NO: 3), QGPPLMWLQV (SEQ ID NO: 4), QGPPLMWTQS (SEQ ID NO: 5), QGPPLMWLQT (SEQ ID NO: 6), QGPPLMWTQV (SEQ ID NO: 7), QGPPLMWMQS (SEQ ID NO: 8), QGPPLMATQS (SEQ ID NO: 9), QGPPLMWLQS (SEQ ID NO: 10), QGPPLMALQV (SEQ ID NO: 11), QGPPLMWLGG (SEQ ID NO: 12), QGPPLMWRGS (SEQ ID NO: 13), QGPLLMWLQV (SEQ ID NO: 14), QGPPLMQTTP (SEQ ID NO: 15), QGPPLSWLQV (SEQ ID NO: 30), QGPPLSWLQS (SEQ ID NO: 31), QGPPGQWSQV (SEQ ID NO: 32), QGPPMMAGLS (SEQ ID NO: 33), QGPPLSWQQS (SEQ ID NO: 34), QGPPGMWSQS (SEQ ID NO: 35), QGPPLQWRQS (SEQ ID NO: 36), QGPPLMGTQS (SEQ ID NO: 37), QGPPLMQLQV (SEQ ID NO: 38), QGPPLSWSQV (SEQ ID NO: 39), QGPPMSWSQS (SEQ ID NO: 40), QGPPLMNLQV (SEQ ID NO: 41), QGPPMSAYQV (SEQ ID NO: 42) und QGPPMQGGLS (SEQ ID NO: 43).

12. Der CCRS-Inhibitor zur Verwendung nach Anspruch 1, wobei die Signatursequenz ausgewählt ist aus der Gruppe QGPPLMALQS (SEQ ID NO: 2), QGPPLMWMQV (SEQ ID NO: 3), QGPPLMWLQV (SEQ ID NO: 4), QGPPLMWTQS (SEQ ID NO: 5), QGPPLMWLQT (SEQ ID NO: 6), QGPPLMWTQV (SEQ ID NO: 7), QGPPLMWMQS (SEQ ID NO: 8), QGPPLMATQS (SEQ ID NO: 9), QGPPLMWLQS (SEQ ID NO: 10), QGPPLMALQV (SEQ ID NO: 11), QGPPLMWLGG (SEQ ID NO: 12), QGPPLMWRGS (SEQ ID NO: 13), QGPLLMWLQV (SEQ ID NO: 14) und QGPPLMQTTP (SEQ ID NO: 15).

13. Der CCRS-Inhibitor zur Verwendung nach Anspruch 1, wobei die Signatursequenz QGPPLMATQS (SEQ ID NO: 9) ist.

14. Der CCRS-Inhibitor zur Verwendung nach Anspruch 13, wobei sich die Signatursequenz am äußersten N-Terminus befindet.

15. Der CCRS-Inhibitor zur Verwendung nach Anspruch 14, wobei der C-terminale Teil mit SEQ ID NO: 1 identisch ist.

16. Der CCRS-Inhibitor zur Verwendung nach Anspruch 1, wobei der CCRS-Inhibitor die Aminosäuresequenz von SEQ ID NO: 70 umfasst.

17. Der CCRS-Inhibitor zur Verwendung nach einem der Ansprüche 1 bis 16, wobei der Krebs kolorektaler Krebs, Brustkrebs, Lungenkrebs, Prostatakrebs, Eierstockkrebs, Bauchspeicheldrüsenkrebs, Speiseröhrenkrebs, Magenkrebs, Leberkrebs oder Leukämie ist.

18. Der CCRS-Inhibitor zur Verwendung nach Anspruch 17, wobei der Krebs metastasiert ist.

19. Der CCR5-Inhibitor zur Verwendung nach Anspruch 17, wobei der Krebs refraktär und/oder resistent gegen Chemotherapie oder Bestrahlung ist.

## Revendications

1. Un inhibiteur de CCR5 pour utilisation dans le traitement du cancer chez un sujet, dans lequel l'inhibiteur de CCR5 comprend une partie N-terminale et une partie C-terminale, où la partie N-terminale comprend la séquence de signature QGP[P ou L] et la séquence d'acides aminés de la partie C-terminale est identique à au moins 70 % à SEQ ID NO : 1, et où l'inhibiteur de CCR5 inhibe l'entrée du VIH dans les cellules et est sélectif pour le CCR5 par rapport à CCR1 et CCR3.

2. L'inhibiteur de CCR5 pour l'utilisation selon la revendication 1, dans lequel l'inhibiteur de CCR5 inhibe qu'un sous-ensemble des voies de signalisation intracellulaires de CCR5.

3. L'inhibiteur de CCR5 pour l'utilisation selon la revendication 1, dans lequel l'inhibiteur de CCR5 inhibe ou réduit les effets inflammatoires de CCR5, par exemple, provoque une réponse de signalisation de 30 % ou moins de la réponse maximale (Emax) déclenchée par PSC-RANTES lorsqu'il est testé à une concentration de 300 nM dans un test de signalisation de flux de calcium.

4. L'inhibiteur de CCR5 pour l'utilisation selon la revendication 1, dans lequel la séquence de signature est QGP[P ou L] [L ou G ou S ou M] [M ou D ou S ou Q ou G].

5. L'inhibiteur de CCR5 pour l'utilisation selon la revendication 1, dans lequel la séquence de signature est QGP[P ou L][L ou G ou S ou M][M ou D ou S ou Q ou G]XX[Q ou G ou L ou A ou T ou S]X, où X désigne tout acide aminé naturel ou modifié.

6. L'inhibiteur de CCR5 pour l'utilisation selon la revendication 1, dans lequel la séquence de signature est QGP[P ou L]LM ou QGPPG[D ou S].

7. L'inhibiteur de CCR5 pour l'utilisation selon la revendication 1, dans lequel la séquence de signature est QGPPLM ou QGPPGD.

8. L'inhibiteur de CCR5 pour l'utilisation selon la revendication 1, dans lequel la séquence de signature est QGP[P ou L][L ou M][M ou Q][A ou W ou G ou Q ou N]X[Q ou G ou L][S ou V ou T ou G], où X désigne tout acide aminé naturel ou modifié.

9. L'inhibiteur de CCR5 pour l'utilisation selon la revendication 1, dans lequel la séquence de signature est QGPPLM[A ou W][L ou T ou M][Q ou G][S ou V ou T ou G].

10. L'inhibiteur de CCR5 pour l'utilisation selon la revendication 1, dans lequel la séquence de signature est QGPP[G ou L][M ou Q]XX[Q ou S][S ou V], où X désigne n'importe quel acide aminé naturel ou modifié.

11. L'inhibiteur de CCR5 pour l'utilisation selon la revendication 1, dans lequel la séquence de signature est sélectionnée dans le groupe QGPPLMALQS (SEQ ID NO: 2), QGPPLMWMQV (SEQ ID NO: 3), QGPPLMWLQV (SEQ ID NO: 4), QGPPLMWTQS (SEQ ID NO: 5), QGPPLMWLQT (SEQ ID NO: 6), QGPPLMWLQV (SEQ ID NO: 7), QGPPLMWMQS (SEQ ID NO: 8), QGPPLMATQS (SEQ ID NO: 9), QGPPLMWLQS (SEQ ID NO: 10), QGPPLMALQV (SEQ ID NO: 11), QGPPLMWLGG (SEQ ID NO: 12), QGPPLMWRGS (SEQ ID NO: 13), QGPLLMWLQV (SEQ ID NO: 14), QGPPLMQTTP (SEQ ID NO: 15), QGPPLSWLQV (SEQ ID NO: 30), QGPPLSWLQS (SEQ ID NO: 31), QGPPGQWSQV (SEQ ID NO: 32), QGPPMMAGLS (SEQ ID NO: 33), QGPPLSWQQS (SEQ ID NO: 34), QGPPGMWSQS (SEQ ID NO: 35), QGPPLQWRQS (SEQ ID NO: 36), QGPPLMGTQS (SEQ ID NO: 37), QGPPLMQLQV (SEQ ID NO: 38), QGPPLSWSQV (SEQ ID NO: 39), QGPPMSWSQS (SEQ ID NO: 40), QGPPLMNLQV (SEQ ID NO: 41), QGPPMSAYQV (SEQ ID NO: 42) et QGPPMQGGLS (SEQ ID NO: 43).

12. L'inhibiteur de CCR5 pour l'utilisation selon la revendication 1, dans lequel la séquence de signature est sélectionnée dans le groupe QGPPLMALQS (SEQ ID NO: 2), QGPPLMWMQV (SEQ ID NO: 3), QGPPLMWLQV (SEQ ID NO: 4), QGPPLMWTQS (SEQ ID NO: 5), QGPPLMWLQT (SEQ ID NO: 6), QGPPLMWTQV (SEQ ID NO: 7), QGPPLMWMQS (SEQ ID NO: 8), QGPPLMATQS (SEQ ID NO: 9), QGPPLMWLQS (SEQ ID NO: 10), QGPPLMALQV (SEQ ID NO: 11), QGPPLMWLGG (SEQ ID NO: 12), QGPPLMWRGS (SEQ ID NO: 13), QGPLLMWLQV (SEQ ID NO: 14) et QGPPLMQTTP (SEQ ID NO: 15).

13. L'inhibiteur de CCR5 pour l'utilisation selon la revendication 1, dans lequel la séquence de signature est QGPPLMATQS (SEQ ID NO: 9).

14. L'inhibiteur de CCR5 pour l'utilisation selon la revendication 13, dans lequel la séquence de signature est située à l'extrémité N-terminale la plus externe.

15. L'inhibiteur de CCR5 pour l'utilisation selon la revendication 14, dans lequel la partie C-terminale est identique à SEQ ID NO : 1.

16. L'inhibiteur de CCR5 pour l'utilisation selon la revendication 1, où l'inhibiteur de CCR5 comprend la séquence d'acides aminés de SEQ ID NO : 70.

17. L'inhibiteur de CCR5 pour l'utilisation selon l'une quelconque des revendications 1 à 16, où le cancer est un cancer colorectal, un cancer du sein, un cancer du poumon, un cancer de la prostate, un cancer de l'ovaire, un cancer du pancréas, un cancer de l'oesophage, un cancer de l'estomac, un cancer du foie, ou une leucémie.

18. L'inhibiteur de CCR5 pour l'utilisation selon la revendication 17, où le cancer est métastasé.

19. L'inhibiteur de CCR5 pour l'utilisation selon la revendication 17, où le cancer est réfractaire et/ou résistant à la chimiothérapie ou à la radiothérapie.
